# EUROPEAN PATENT SPECIFICATION

(11) **EP 1 910 357 B1**
(45) Date of publication and mention of the grant of the patent: **14.04.2010**
(21) Application number: 06755627.4
(22) Date of filing: 26.06.2006
(51) Int. Cl.: C07D 471/04

(54) **INDOLI ZINE DERIVATIVES AND THEIR USE AS CRTH2 ANTAGONISTS**
INDOLIZINDERIVATE UND DEREN VERWENDUNG ALS CRTH2-ANTAGONISTEN
DÉRIVÉS D'INDOLIZINE ET LEUR UTILISATION COMME ANTAGONISTES DU CRTH2

(30) Priority: 24.06.2005 GB 0512944
(43) Date of publication of application: 16.04.2008
(73) Proprietor: Argenta Discovery Limited, Harlow, Essex CM19 5TR (GB)
(72) Inventor: HYND, George, Harlow Essex CM19 5TR (GB); RAY, Nicholas, Charles, Harlow Essex CM19 5TR (GB); FINCH, Harry, Harlow Essex CM19 5TR (GB); MIDDLEMISS, David, Bishps Stortford, Hertfordshire (GB); CRAMP, Michael, Colin, Harlow Essex CM19 5TR (GB); BLANEY, Paul, Matthew, Harlow Essex CM19 5TR (GB); WILLIAMS, Karen, Harlow Essex CM19 5TR (GB); GRIFFON, Yan A, Harlow Essex CM19 5TR (GB); HARRISON, Trevor Keith, Harlow Essex CM19 5TR (GB); CRACKETT, Peter, Harlow Essex CM19 5TR (GB)
(74) Representative: Perry, Robert Edward
(86) International application number: PCT/GB2006/002341
(87) International publication number: WO 2006/136859

(56) References cited:
- WO-A-2005/040112
- WO-A-2005/040114
- WO-A-2005/044260
- ES-A1- 421 284
- GB-A- 1 174 124
- GB-A- 1 268 424
- GB-A- 2 407 318
- "THE USE OF INDOLE-3-ACETIC ACIDS AS CRTH2 RECEPTOR ANTAGONISTS" EXPERT OPINION ON THERAPEUTIC PATENTS, ASHLEY PUBLICATIONS, GB, vol. 14, no. 1, 2004, pages 125-128, XP009051302 ISSN: 1354-3776
- HATA A N ET AL: "STRUCTURAL DETERMINANTS OF ARYLACETIC ACID NONSTEROIDAL ANTI-INFLAMMATORY DRUGS NECESSARY FOR BINDING AND ACTIVATION OF THE PROSTAGLANDIN D2 RECEPTOR CRTH2" MOLECULAR PHARMACOLOGY, BALTIMORE, MD, US, vol. 67, no. 3, March 2005 (2005-03), pages 640-647, XP009051298 ISSN: 0026-895X
- CASAGRANDE C ET AL: "INDOLIZINE ANALOGUES OF INDOMETHACIN" FARMACO, EDIZIONE SCIENTIFICA, SOCIETA CHIMICA ITALIANA, PAVIA, IT, vol. 26, 1971, pages 1059-1073, XP009074694 ISSN: 0430-0920

## Description

This invention relates to a class of indolizine compounds which are ligands of the CRTH2 receptor (Chemoattractant Receptor-homologous molecule expressed on T Helper cells type 2), and their use in the treatment of diseases responsive to modulation of CRTH2 receptor activity, principally diseases having a significant inflammatory component. The invention also relates to novel members of that class of ligands and pharmaceutical compositions containing them.

### Background to the Invention

Mast cells are known to play an important role in allergic and immune responses through the release of a number of mediators, such as histamine, leukotrienes, cytokines, prostaglandin D₂, etc (Boyce; Allergy Asthma Proc., 2004, 25, 27-30). Prostaglandin D₂ (PGD₂) is the major metabolite produced by the action of cyclooxygenase on arachadonic acid by mast cells in response to allergen challenge (Lewis et al; J. Immunol., 1982, 129, 1627-1631). It has been shown that PGD₂ production is increased in patients with systemic mastocytosis (Roberts; N. Engl. J. Med., 1980, 303, 1400-1404), allergic rhinitis (Naclerio et al; Am. Rev. Respir. Dis., 1983, 128, 597-602; Brown et al; Arch. Otolarynol. Head Neck Surg., 1987, 113, 179-183; Lebel et al; J. Allergy Clin. Immunol., 1988, 82, 869-877), bronchial asthma (Murray et al; N. Engl. J. Med., 1986, 315, 800-804; Liu et al; Am. Rev. Respir. Dis., 1990, 142, 126-132; Wenzel et al; J. Allergy Clin. Immunol., 1991, 87, 540-548), and urticaria (Heavey et al; J. Allergy Clin. Immunol., 1986, 78, 458-461). PGD₂ mediates it effects through two receptors, the PGD₂ (or DP) receptor (Boie et al; J. Biol. Chem., 1995, 270, 18910-18916) and the chemoattractant receptor-homologous molecule expressed on Th2 (or CRTH2) (Nagata et al; J. Immunol., 1999, 162, 1278-1289; Powell; Prostaglandins Luekot. Essent. Fatty Acids, 2003, 69, 179-185). Therefore, it has been postulated that agents that antagonise the effects of PGD₂ at its receptors may have beneficial effects in number of disease states.

The CRTH2 receptor has been shown to be expressed on cell types associated with allergic inflammation, such as basophils, eosinophils, and Th2-type immune helper cells (Hirai et al; J. Exp. Med., 2001, 193, 255-261). The CRTH2 receptor has been shown to mediate PGD₂-mediated cell migration in these cell types (Hirai et al; J. Exp. Med., 2001, 193, 255-261), and also to play a major role in neutrophil and eosinophil cell recruitment in a model of contact dermatitis (Takeshita et al; Int. Immunol., 2004, 16, 947-959). Ramatroban {(3R)-3-[(4-fluorophenyl)sulphonylamino]-1,2,3,4-tetrahydro-9H-carbazole-9-propanoic acid}, a dual CRTH2 and thromboxane A₂ receptor antagonist, has been shown to attenuate these responses (Sugimoto et al; J. Pharmacol. Exp. Ther., 2003, 305, 347-352; Takeshita et al; *op. cit.*)*.* The potential of PGD₂ both to enhance allergic inflammation and induce an inflammatory response has been demonstrated in mice and rats. Transgenic mice over expressing PGD₂ synthase exhibit an enhanced pulmonary eosinophilia and increased levels of Th2 cytokines in response to allergen challenge (Fujitani et al, J. Immunol., 2002, 168, 443-449). In addition, exogenously administered CRTH2 agonists enhance the allergic response in sensitised mice (Spik et al; J. Immunol., 2005, 174, 3703-3708). In rats exogenously applied CRTH2 agonists cause a pulmonary eosinophilia but a DP agonist (BW 245C) or a TP agonist (I-BOP) showed no effect (Shirashi et al; J. Pharmacol. Exp. Ther., 2005, 312, 954-960). These observations suggest that CRTH2 antagonists may have valuable properties for the treatment of diseases mediated by PGD₂.

In addition to ramatroban a number of other CRTH2 antagonists have been described. Examples include: indole-acetic acids (WO2003/022813; WO2003/066046; WO2003/066047; WO2003/097042; WO2003/097598; WO2003/101961; W02003/101981; W02004/007451; WO2004/078719; WO2004/106302; WO2005/019171; GB2407318; W02005/040112; W02005/040114; WO2005/044260); tetrahydroquinolines (EP1413306; EP1435356; WO2004/032848; WO2004/035543; WO2005/007094), and phenylacetic acids (WO2004/058164; WO2004/089884; WO2004/089885; WO2005/018529).

ES 421284 relates to indolizine derivatives which are claimed to be analgesic and anti-inflammatory agents. DE 2046904 and GB 1174124 also relate to indolizine derivatives. Malonne, H. et al. Pharmacy and Pharmacology Communications 1998, 4, 241-243, Rosseels, G. et al. Eur. J. Med Chem. 1975, 10, 579-584 and Casagrande, C. et al. Farmaco 1971, 26, 1059-1073 refer to indolizine compounds having anti-inflammatory activity.

### Detailed Description of the Invention

According to the present invention, there is provided a compound of formula (I) or a salt, N-oxide, hydrate or solvate thereof: 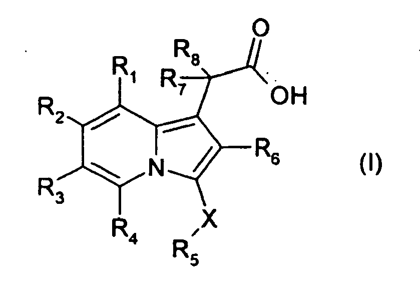 wherein
**R₁, R₂, R₃** and **R₄** each independently are hydrogen, C₁-C₆alkyl, fully or partially fluorinated C₁-C₆alkyl, halo, -S(O)ₙR₁₀, -SO₂NR₁₀)₂, -N(R₁₀)₂, -C(O)N(R₁₀)₂,-NR₁₀C(O)R₉, -CO₂R₁₀,-C(O)R₉, -NO₂, -CN or -OR₁₁;
wherein each **R₉** is independently methyl, ethyl or phenyl;
**R₁₀** is independently hydrogen, methyl, ethyl or phenyl;
**R₁₁** is hydrogen, C₁-C₆alkyl, fully or partially fluorinated C₁-C₆alkyl, or -SO₂R₉; and
n is 0, 1 or 2;
**R₅** is C₁-C₆alkyl, fully or partially fluorinated C₁-C₆alkyl, C₁-C₆alkenyl, C₁-C₆alkynyl, optionally substituted phenyl, or optionally substituted monocyclic heteroaryl having 5 or 6 ring atoms;
**R₆** is hydrogen, C₁-C₆alkyl or fully or partially fluorinated C₁-C₆alkyl;
either **R₇** and **R₈** are independently hydrogen or C₁-C₆alkyl, or **R₇** and **R₈** together with the atom to which they are attached form a cycloalkyl group; and
X is -CHR₆-, -S(O)ₙ-, -NR₆SO₂- or -SO₂NR₆-, wherein n is 0, 1 or 2.

One aspect of the invention is these compounds for therapeutic use. Another aspect is these compounds, independent of use, with the proviso that, when X is -CH₂-, R₆ is methyl and R₅ is 4-chlorophenyl, then R₁, R₂, R₃, R₄, R₇ and R₈ are not all hydrogen. This proviso excludes a compound referred to in Casagrande et al, II Farmaco - Ed. Sc. - Vol 26, pp 1059-1073, to which no pharmaceutical utility is ascribed.

Compounds (I) with which the invention is concerned are CRTH2 receptor antagonists, but they may also have beneficial effects at other prostanoid receptors, such as the PGD₂ receptor or the thromboxane A₂ receptor.

A third aspect of the invention is a pharmaceutical composition comprising a compound of formula (I), or a salt, N-oxide, hydrate or solvate thereof, in admixture with a pharmaceutically acceptable carrier or excipient.

A fourth aspect of the invention is the use of a compound of formula (I), or a salt, N-oxide, hydrate or solvate thereof, in the manufacture of a medicament for the treatment of a disease in which a CRTH2 antagonist can prevent, inhibit or ameliorate the pathology and/or symptomatology of the disease.

### Description of Preferred Embodiments

In particular, compounds with which the invention is concerned are useful in the treatment of disease associated with elevated levels of prostaglandin D2 (PGD2) or one or more active metabolites thereof.

Examples of such diseases include asthma, rhinitis, allergic airway syndrome, allergic rhinobronchitis, bronchitis, chronic obstructive pulmonary disease (COPD), nasal polyposis, sarcoidosis, farmer's lung, fibroid lung, cystic fibrosis, chronic cough, conjunctivitis, atopic dermatitis, Alzheimer's disease, amyotrophic lateral sclerosis, AIDS dementia complex, Huntington's disease, frontotemporal dementia, Lewy body dementia, vascular dementia, Guillain-Barre syndrome, chronic demyelinating polyradiculoneurophathy, multifocal motor neuropathy, plexopathy, multiple sclerosis, encephalomyelitis, panencephalitis, cerebellar degeneration and encephalomyelitis, CNS trauma, migraine, stroke, rheumatoid arthritis, ankylosing spondylitis, Behçet's Disease, bursitis, carpal tunnel syndrome, inflammatory bowel disease, Crohn's disease, ulcerative colitis, dermatomyositis, Ehlers-Danlos Syndrome (EDS), fibromyalgia, myofascial pain, osteoarthritis (OA), osteonecrosis, psoriatic arthritis, Reiter's syndrome (reactive arthritis), sarcoidosis, scleroderma, Sjogren's Syndrome, soft tissue disease, Still's Disease, tendinitis, polyarteritis Nodossa, Wegener's Granulomatosis, myositis (polymyositis dermatomyositis), gout, atherosclerosis, lupus erythematosus, systemic lupus erythematosus (SLE), type I diabetes, nephritic syndrome, glomerulonephritis, acute and chronic renal failure, eosinophilia fascitis, hyper IgE syndrome, sepsis, septic shock, ischemic reperfusion injury in the heart, allograft rejection after transplantations, and graft versus host disease.

However, the compounds with which the invention is concerned are primarily of value for the treatment of asthma, chronic obstructive pulmonary disease, rhinitis, allergic airway syndrome, and allergic rhinobronchitis.

### Terminology

As used herein, the term "(Cₐ-C_{b})alkyl" wherein a and b are integers refers to a straight or branched chain alkyl radical having from a to b carbon atoms. Thus when a is 1 and b is 6, for example, the term includes methyl, ethyl, n-propyl, isopropyl, n-butyl, isobutyl, sec-butyl, t-butyl, n-pentyl and n-hexyl.

As used herein, the term "fully or partially fluorinated Cₐ-C_{b}alkyl" wherein a and b are integers refers to a straight or branched chain alkyl radical having from a to b carbon atoms in which the hydrogen atoms all replaced by fluorine (fully fluorinated) or in which some of the hydrogen atoms are rfeplaced by fluorine (partially fluorinated). The term includes, for example -CF₃, -CHF₂, -CFH₂, and CF₃CH₂-.

As used herein the term "(Cₐ-C_{b})alkenyl" wherein a and b are integers refers to a straight or branched chain alkenyl moiety having from a to b carbon atoms having at least one double bond of either E or Z stereochemistry where applicable. The term includes, for example, vinyl, allyl, 1- and 2-butenyl and 2-methyl-2-propenyl.

As used herein the term "Cₐ-C_{b} alkynyl" wherein a and b are integers refers to straight chain or branched chain hydrocarbon groups having from two to six carbon atoms and having in addition one triple bond. This term would include for example, ethynyl, 1- and 2-propynyl, 1-, 2- and 3-butynyl, 1, 2-, 3- and 4-pentynyl, 1-, 2-, 3-, 4- and 5-hexynyl, 3-methyl-1-butynyl, 1-methyl-2-pentynyl.

As used herein the term "carbocyclic" refers to a mono-, bi- or tricyclic radical having up to 16 ring atoms, all of which are carbon, and includes aryl and cycloalkyl.

As used herein the term "cycloalkyl" refers to a monocyclic saturated carbocyclic radical having from 3-8 carbon atoms and includes, for example, cyclopropyl, cyclobutyl, cyclopentyl, cyclohexyl, cycloheptyl and cyclooctyl.

As used herein the unqualified term "aryl" refers to a mono-, bi- or tri-cyclic carbocyclic aromatic radical, and includes radicals having two monocyclic carbocyclic aromatic rings which are directly linked by a covalent bond. Illustrative of such radicals are phenyl, biphenyl and napthyl.

As used herein the unqualified term "heteroaryl" refers to a mono-, bi- or tri-cyclic aromatic radical containing one or more heteroatoms selected from S, N and O, and includes radicals having two such monocyclic rings, or one such monocyclic ring and one monocyclic aryl ring, which are directly linked by a covalent bond. Illustrative of such radicals are thienyl, benzthienyl, furyl, benzfuryl, pyrrolyl, imidazolyl, benzimidazolyl, thiazolyl, benzthiazolyl, isothiazolyl, benzisothiazolyl, pyrazolyl, oxazolyl, benzoxazolyl, isoxazolyl, benzisoxazolyl, isothiazolyl, triazolyl, benztriazolyl, thiadiazolyl, oxadiazolyl, pyridinyl, pyridazinyl, pyrimidinyl, pyridazinyl, triazinyl, indolyl and indazolyl.

As used herein the unqualified term "heterocyclyl" or "heterocyclic" includes "heteroaryl" as defined above, and in addition means a mono-, bi- or tri-cyclic non-aromatic radical containing one or more heteroatoms selected from S, N and O, and to groups consisting of a monocyclic non-aromatic radical containing one or more such heteroatoms which is covalently linked to another such radical or to a monocyclic carbocyclic radical. Illustrative of such radicals are pyrrolyl, furanyl, thienyl, piperidinyl, imidazolyl, oxazolyl, isoxazolyl, thiazolyl, thiadiazolyl, pyrazolyl, pyridinyl, pyrrolidinyl, pyrimidinyl, morpholinyl, piperazinyl, indolyl, quinolyl, morpholinyl, benzfuranyl, pyranyl, isoxazolyl, benzimidazolyl, methylenedioxyphenyl, ethylenedioxyphenyl, maleimido and succinimido groups.

Unless otherwise specified in the context in which it occurs, the term "substituted" as applied to any moiety herein means substituted with up to four compatible substituents, each of which independently may be, for example, (C₁-C₆)alkyl, cycloalkyl, (C₁-C₆)alkoxy, hydroxy, hydroxy(C₁-C₆)alkyl, mercapto, mercapto(C₁-C₆)alkyl, (C₁-C₆)alkylthio, phenyl, monocyclic heteroaryl having 5 or 6 ring atoms, halo (including fluoro, bromo and chloro), trifluoromethyl, trifluoromethoxy, nitro, nitrile (-CN), oxo, -COOH, -COOR^{A}, -COR^{A}, -SO₂R^{A}, -CONH₂, -SO₂NH₂ -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A} -NHCOOR^{A}, -NR^{B}COOR^{A}, -NHSO₂OR^{A}, -NR^{B}SO₂OH, -NR^{B}SO₂OR^{A},-NHCONH₂, -NR^{A}CONH₂, -NHCONHR^{B}, -NR^{A}CONHR^{B}, -NHCONR^{A}R^{B}, or -NR^{A}CONR^{A}R^{B} wherein R^{A} and R^{B} are independently a (C₁-C₆)alkyl, (C₃-C₆) cycloalkyl , phenyl or monocyclic Heteroaryl having 5 or 6 ring atoms. An "optional substituent" may be one of the foregoing substituent groups.

As used herein the term "salt" includes base addition, acid addition and quaternary salts. Compounds of the invention which are acidic can form salts, including pharmaceutically acceptable salts, with bases such as alkali metal hydroxides, e.g. sodium and potassium hydroxides; alkaline earth metal hydroxides e.g. calcium, barium and magnesium hydroxides; with organic bases e.g. N-methyl-D-glucamine, choline tris(hydroxymethyl)amino-methane, L-arginine, L-lysine, N-ethyl piperidine, dibenzylamine and the like. Specific salts with bases include the benzathine, calcium, diolamine, meglumine, olamine, potassium, procaine, sodium, tromethamine and zinc salts. Those compounds (I) which are basic can form salts, including pharmaceutically acceptable salts with inorganic acids, e.g. with hydrohalic acids such as hydrochloric or hydrobromic acids, sulphuric acid, nitric acid or phosphoric acid and the like, and with organic acids e.g. with acetic, tartaric, succinic, fumaric, maleic, malic, salicylic, citric, methanesulphonic, p-toluenesulphonic, benzoic, benzenesunfonic, glutamic, lactic, and mandelic acids and the like.

Compounds with which the invention is concerned which may exist in one or more stereoisomeric form, because of the presence of asymmetric atoms or rotational restrictions, can exist as a number of stereoisomers with R or S stereochemistry at each chiral centre or as atropisomeres with R or S stereochemistry at each chiral axis. The invention includes all such enantiomers and diastereoisomers and mixtures thereof.

Use of prodrugs, such as esters, of compounds (I) with which the invention is concerned is also part of the invention. "Prodrug" means a compound which is convertible *in vivo* by metabolic means (e.g. by hydrolysis, reduction or oxidation) to a compound of formula (I). For example an ester prodrug of a compound of formula (I) may be convertible by hydrolysis *in vivo* to the parent molecule. Suitable esters of compounds of formula (I) are for example acetates, citrates, lactates, tartrates, malonates, oxalates, salicylates, propionates, succinates, fumarates, maleates, methylene-bis-β-hydroxynaphthoates, gentisates, isethionates, di-*p*-toluoyltartrates, methanesulphonates, ethanesulphonates, benzenesulphonates, *p*-toluenesulphonates, cyclohexylsulphamates and quinates. Examples of ester prodrugs are those described by F. J. Leinweber, Drug Metab. Res., 1987, 18, 379. As used in herein, references to the compounds of formula (I) are meant to also include the prodrug forms.

### The variables R₁-R₈

For use in accordance with the invention, the following structural characteristics are currently preferred, in any compatible combination, in the compounds (I):
R₁, R₂, R₃ and R₄ are preferably electron-deficient or electron-withdrawing substituents, and may be independently selected from, for example, hydrogen methyl, ethyl, trifluoromethyl, fluoro, chloro, bromo, -NO₂, -CN, -SO₂R₉, -SO₂N(R₁₀)₂, -C(O)N(R₁₀)₂, -NR₁₀C(O)R₉, -CO₂R₁₀, and -C(O)R₉, wherein R₉, R₁₀ and R₁₁ are as defined above with reference to formula (I). Thus: R₉ may be selected from, for example, methyl, ethyl, and phenyl; R₁₀ may be selected from, for example, hydrogen, methyl, ethyl, and phenyl; and R₁₁ may be selected from, for example, methyl, trifluoromethyl, ethyl, phenyl,-SO₂H and -SO₂CH₃. Currently preferred are compounds in which R₁, R₂, R₃ and R₄ are independently selected from hydrogen, chloro, fluoro, cyano, methyl, trifluoromethyl. Often, two of R₁, R₂, R₃ and R₄ be hydrogen, while the others, for example R₂ and R₃, are independently selected from hydrogen, chloro, fluoro, cyano, methyl, and trifluoromethyl.

R₅ may be, for example, methyl, ethyl, n- or iso-propyl, trifluoromethyl, allyl, optionally substititued phenyl or naphthyl; or optionally substituted monocyclic heteroaryl having 5 or 6 ring atoms, such as optionally susbtituted pyridyl, pyrimidinyl, furyl, thienyl, imidazolyl, oxazolyl or isoxazolyl or pyrrolyl; or optionally substituted bicyclic heteroaryl having 8 to 10 ring atoms, such as quinolinyl, indolyl, and benzimidazolyl. Optional substituents in any such ring systems may be any of those listed above with reference to the term "substituted", for example chloro, fluoro, trifluoromethyl, methylsulfonyl, ethylsulfonyl, carbamate, methylcarbamate, methylaminosulfonyl, ethylaminosulfonyl, methylsulfonylamino, ethylsulfonylamino, morpholin-1-ylsulfonyl, piperidin-1-ylsulfonyl, piperizin-1-ylsulfonyl, 4-methylpiperizin-1-ylsulfonyl, and tetrahydropyrrol-1ylsulfonyl.

The divalent radical -X- may be, for example, -CH₂-, -S-, -S(O)-, -SO₂-, -NHSO₂- or -SO₂NH-. Currently it is preferred that -X- be -S- or -SO₂-.

R₆ may be, for example, hydrogen, methyl, ethyl or trifluoromethyl. Currently it is preferred that R₆ be methyl.

R₇ and R₈ may both be hydrogen, or one of R₇ and R₈ may be, for example, methyl whiule the other is hydrogen, or R₇ and R₈ taken together with the carbon atom to which they are attached may form, for example, a cyclopropyl, cyclopentyl or cyclohexyl ring. Presently it is ptreferred that both R₇ and R₈ be hydrogen.

The carboxylic acid group attached to -C(R₇)(R₈)- may be esterified as an ester which is hydrolysed in vivo to release the carboxylic acid, and in such cases the compound is an ester prodrug of compounds (I).

In one particular subclass of the compounds with which the invention is concerned, R₁, R₂, R₃ and R₄ are independently selected from hydrogen, chloro, fluoro, cyano, methyl, and trifluoromethyl; X is -S- or -SO₂-; R₅ is optionally substituted phenyl, R₆ is methyl, and R₇ and R₈ are hydrogen. In this sublass, when R₅ is optionally substituted phenyl, one or two substituents may be present, independently selected from chloro, fluoro, methylsulfonyl, ethylsulfonyl, carbamate, methylcarbamate, methylaminosulfonyl, ethylaminosulfonyl, methylsulfonylamino, ethylsulfonylamino, morpholin-1-ylsulfonyl, piperidin-1-ylsulfonyl, piperizin-1-ylsulfonyl, 4-methylpiperizin-1-ylsulfonyl, and tetrahydropyrrol-1ylsulfonyl.

Specific examples of compounds with which the invention is concerned include those of the examples herein.

### Compositions

As mentioned above, the compounds with which the invention is concerned are CRTH2 receptor antagonists, and are useful in the treatment of diseases which benefit from such modulation. Examples of such diseases are referred to above, and include asthma, rhinitis, allergic airway syndrome, and allergic rhinobronchitis.

It will be understood that the specific dose level for any particular patient will depend upon a variety of factors including the activity of the specific compound employed, the age, body weight, general health, sex, diet, time of administration, route of administration, rate of excretion, drug combination and the severity of the particular disease undergoing treatment. Optimum dose levels and frequency of dosing will be determined by clinical trial, as is required in the pharmaceutical art. In general, the daily dose range will lie within the range of from about 0.001 mg to about 100 mg per kg body weight of a mammal, often 0.01 mg to about 50 mg per kg, for example 0.1 to 10 mg per kg, in single or divided doses. On the other hand, it may be necessary to use dosages outside these limits in some cases.

The compounds with which the invention is concerned may be prepared for administration by any route consistent with their pharmacokinetic properties. Orally administrable compositions may be in the form of tablets, capsules, powders, granules, lozenges, liquid or gel preparations, such as oral, topical, or sterile parenteral solutions or suspensions. Tablets and capsules for oral administration may be in unit dose presentation form, and may contain conventional excipients such as binding agents, for example syrup, acacia, gelatin, sorbitol, tragacanth, or polyvinyl-pyrrolidone; fillers for example lactose, sugar, maize-starch, calcium phosphate, sorbitol or glycine; tabletting lubricant, for examples magnesium stearate, talc, polyethylene glycol or silica; disintegrants for example potato starch, or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, syrup, methyl cellulose, glucose syrup, gelatin hydrogenated edible fats; emulsifying agents, for example lecithin, sorbitan monooleate, or acacia; nonaqueous vehicles (which may include edible oils), for example almond oil, fractionated coconut oil, oily esters such as glycerine, propylene glycol, or ethyl alcohol; preservatives, for example methyl or propyl p-hydroxybenzoate or sorbic acid, and if desired conventional flavouring or colouring agents.

For topical application to the skin, the drug may be made up into a cream, lotion or ointment. Cream or ointment formulations which may be used for the drug are conventional formulations well known in the art, for example as described in standard textbooks of pharmaceutics such as the British Pharmacopoeia.

The drug may also be formulated for inhalation, for example as a nasal spray, or dry powder or aerosol inhalers. For delivery by inhalation, the active compound is preferably in the form of microparticles. They may be prepared by a variety of techniques, including spray-drying, freeze-drying and micronisation. Aerosol generation can be carried out using, for example, pressure-driven jet atomizers or ultrasonic atomizers, preferably using propellant-driven metered aerosols or propellant-free administration of micronized active compounds from, for example, inhalation capsules or other "dry powder" delivery systems.

The active ingredient may also be administered parenterally in a sterile medium. Depending on the vehicle and concentration used, the drug can either be suspended or dissolved in the vehicle. Advantageously, adjuvants such as a local anaesthetic, preservative and buffering agents can be dissolved in the vehicle.

Other compounds may be combined with compounds of this invention of formula [I] for the prevention and treatment of prostaglandin-mediated diseases. Thus the present invention is also concerned with pharmaceutical compositions for preventing and treating PGD₂-mediated diseases comprising a therapeutically effective amount of a compound of the invention of formula [I] and one or more other therapeutic agents. Suitable therapeutic agents for a combination therapy with compounds of formula [I] include, but are not limited to: (1) corticosteroids, such as fluticasone, budesonide or ciclesonide; (2) β2-adrenoreceptor agonists, such as salmeterol, formeterol or indacaterol; (3) leukotriene modulators, for example leukotriene antagonists such as montelukast or pranlukast or leukotriene biosynthesis inhibitors such as Zileuton or BAY-x1005; (4) anticholinergic agents, for example muscarinic-3 (M₃) receptor antagonists such as tiotropium bromide; (5) phosphodiesterase-IV (PDE-IV) inhibitors, such as roflumilast or cilomilast; (6) antihistamines, for example selective histamine-1 (H1) receptor antagonists, such as loratidine or astemizole; (7) antitussive agents, such as codeine or dextramorphan; (8) non-selective COX-1 /COX-2 inhibitors, such as ibuprofen or ketoprofen; (9) COX-2 inhibitors, such as celecoxib and rofecoxib; (10) VLA-4 antagonists, such as those described in WO97/0309 and WO97/02289; (11) TNF-α inhibitors, for example anti-TNF monoclonal antibodies, such as Remicade and CDP-870 and TNF receptor immunoglobulin molecules, such as Enbrel; (12) inhibitors of matrix metalloprotease (MMP), for example MMP8, 9 and 12; (13) human neutrophil elastase inhibitors, such as those described in WO2005/026124 and WO2003/053930; (14) Adenosine A2a agonists such as those described in EP1052264 and EP1241176 (15) Adenosine A2b antagonists such as those described in WO2002/42298; (16) modulators of chemokine receptor function, for example antagonists of CCR3 and CCR8; (17) compounds which modulate the action of other prostanoid receptors, for example a PGD2 (DP) receptor antagonist or a thromboxane A2 antagonist; and (18) compounds which modulate Th2 function, for example, PPAR agonists.

The weight ratio of the compound of the invention to the second active ingredient may be varied and will depend upon the effective dose of each ingredient. Generally, an effective dose of each will be used.

### Methods of Synthesis

The present invention is also concerned with processes for preparing the compounds of this invention.

The compounds of the present invention can be prepared according to the procedures of the following schemes and examples, using appropriate materials, and are further exemplified by the following specific examples. Moreover, by utilizing the procedures described with the disclosure contained herein, one of ordinary skill in the art can readily prepare additional compounds of the present invention claimed herein. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds.

The compounds of the invention may be isolated in the form of their pharmaceutically acceptable salts, such as those described previously herein above. The free acid form corresponding to isolated salts can be generated by acidification with a suitable acid such as acetic acid and hydrochloric acid and extraction of the liberated free acid into an organic solvent followed by evaporation. The free acid form isolated in this manner can be further converted into another pharmaceutically acceptable salt by dissolution in an organic solvent followed by addition of the appropriate base and subsequent evaporation, precipitation, or crystallisation.

It may be necessary to protect reactive functional groups (e.g. hydroxy, amino, thio or carboxy) in intermediates used in the preparation of compounds of the invention to avoid their unwanted participation in a reaction leading to the formation of the desired compounds. Conventional protecting groups, for example those described by T. W. Greene and P. G. M. Wuts in "Protective groups in organic chemistry" John Wiley and Sons, 1999, may be used.

Compounds of the invention of formula [1a] in which group R⁵ is attached to the indolizine ring through a thio group, may conveniently be prepared from an intermediate compound of formula [2] with a disulfide compound of formula [9], in a solvent such as an alcohol, for example methanol, at the reflux temperature of the solvent, followed by deprotection of the ester group R¹² to give the desired carboxylic acid of formula [1 a]. Alternatively a sulfenyl halide may be used in place of the disulfide [9]. Compounds of Formula [9] are commercially available or may be prepared by known methods.

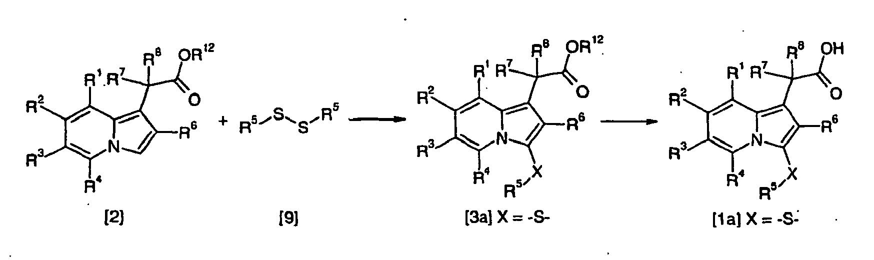

Intermediate compounds of formula [2], may conveniently be prepared by the reaction between a compound of formula [4] and a suitable alkylating agent of formula [5], where group LG represents a suitable leaving group (for example, chloro, bromo, or methanesulfonyloxy). Typically, the alkylation reaction is carried out in the presence of a base (for example, sodium hydrogen carbonate) in an inert solvent (for example, 2-butanone) or acetonitrile, at the reflux temperature of the solvent.

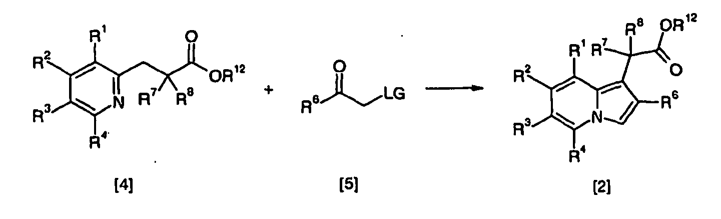

Compounds of formula [4], in which R⁸ is hydrogen, may be prepared by the reaction of pyridine-2-carboxaldehydes of formula [6] with an appropriate malonic acid, followed by protection of the carboxylic acid group and reduction of intermediate compounds of formula [8]. The transformation of compounds of formula [8] to those of formula [4] may conveniently be achieved by reduction with hydrogen in the presence of a suitable catalyst, such as palladium supported on carbon.

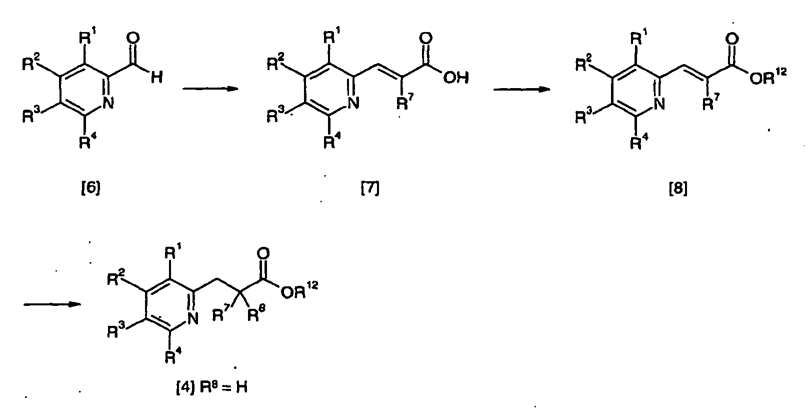

Alternatively, compounds of Formula [4] may be prepared from compounds of Formula [10] by reaction with an organozinc reagent of Formula [11]. The reaction my be catalysed by a palladium catalys, for example, Pd(PPh₃)₄, in a solvent such as THF.

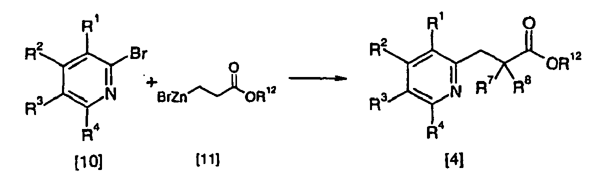

It will be understood by those practiced in the art that compounds of the invention may be prepared by transformations of other compounds of the invention. For example, compounds of the invention of formula [1b], in which group X represents a sulfonyl group, may conveniently be prepared by the oxidation of compounds of the invention of formula [1a], with a suitable oxidising agent such as potassium peroxymonosulfate, *meta*-chloroperoxybenzoic acid or other well known oxidising agents.

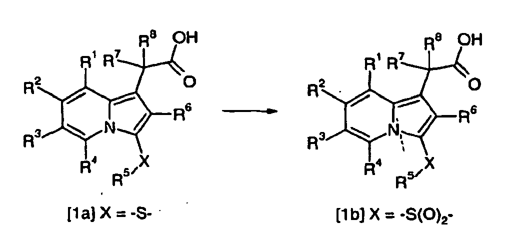

Compounds of Formula [1c] where X is a -CH₂- may be prepared from compounds of Formula [2] by reaction with a suitable alkyl halide of Formula [12], such as a benzyl halide. Compounds of Formula [12] are well-known in the literature or are commercially available.

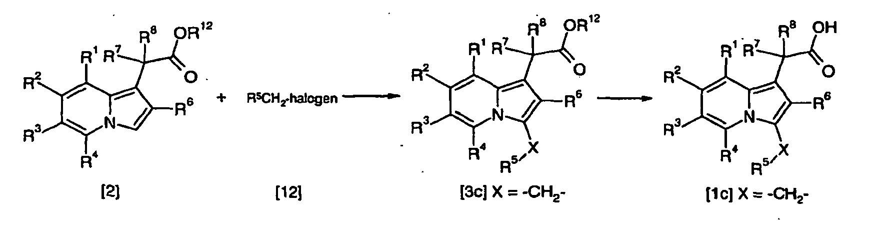

Compounds of Formula [1d] where X is a -SO₂NR- group may be prepared from compounds of Formula [2] by treatment with a suitably substituted sulfamoyl halide of Formula [13]. Sulfamoyl halides are readily prepared from the corresponding amines by known methods, and such amines are commercially available or known in the literature.

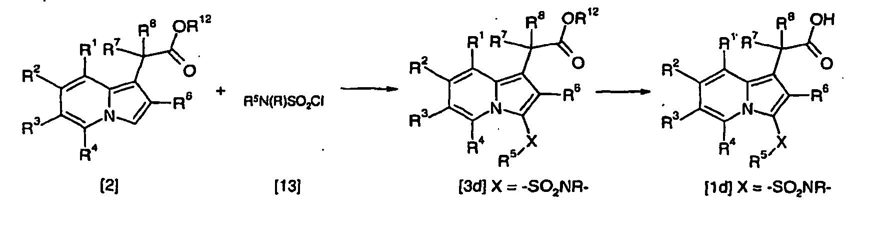

### Biological Methods

Compounds of the invention can be tested using the following biological test methods to determine their ability to displace PGD₂ from the CRTH2 receptor and for their ability to antagonise the functional effects of PGD₂ at the CRTH2 receptor in a whole cell system.

### Radioligand Binding Assay

The receptor binding assay is performed in a final volume of 200 µL binding buffer [10 mM BES (pH 7.4), 1 mM EDTA, 10 mM manganese chloride, 0.01 % BSA] and 1 nM [³H]-PGD₂ (Amersham Biosciences UK Ltd). Ligands are added in assay buffer containing a constant amount of DMSO (1 % by volume). Total binding is determined using 1 % by volume of DMSO in assay buffer and non-specific binding is determined using 10 µM of unlabeled PGD₂ (Sigma). Human embryonic kidney (HEK) cell membranes (3.5 µg) expressing the CRTH2 receptor are incubated with 1.5 mg wheatgerm agglutinin SPA beads and 1 nM [³H]-PGD₂ (Amersham Biosciences UK Ltd) and the mixture incubated for 3 hours at room temperature. Bound [³H]-PGD₂ is detected using a Microbeta TRILUX liquid scintillation counter (Perkin Elmer). Compound IC₅₀ value is determined using a 6-point dose response curve in duplicate with a semi-log compound dilution series. IC₅₀ calculations are performed using Excel and XLfit (Microsoft), and this value is used to determine a Ki value for the test compound using the Cheng-Prusoff equation.

### Functional Assays

GTPγS Assay

The GTPγS Assay is performed in a final volume of 200 mL assay buffer (20mM HEPES pH 7.4, 10mM MgCl₂, 100mM NaCl, 10µ/mL saponin). DMSO concentrations are kept constant at 1% by volume. Human embryonic kidney (HEK) cell membranes (3.5 µg) expressing the CRTH2 receptor are incubated with the compounds for 15 min at 30° C prior to addition of PGD₂ (30nM final concentration) and GTP (10nM final concentration). The assay solutions are then incubated for 30 minutes at 30° C, followed by addition of [35S]-GTPγS (0.1 nM final concentration). The assay plate is than shaken and incubated for 5 minutes at 30°C. Finally, SPA beads (Amersham Biosciences, UK) are added to a final concentration of 1.5mg/well and the plate shaken and incubated for 30 minute at 30° C. The sealed plate is centrifuged at 1000g for 10mins at 30 °C and the bound [³⁵S]-GTPγS is detected on Microbeta scintillation counter (Perkin Elmer). Compound IC₅₀ value is determined using a 6-point dose response curve in duplicate with a semi-log compound dilution series. IC₅₀ calculations are performed using Excel and XLfit (Microsoft), and this value is used to determine a Ki value for the test compound using the Cheng-Prusoff equation.

### Calcium mobilisation assay

Stable CHO-K1 cells co-expressing the CRTH2 receptor and the G-protein Gα16 are seeded (40,000 cells per well in a plating volume of 75 µL in F-12 Hams supplemented with 1 % foetal bovine serum) into collagen-coated 96-well plates 24 hours prior to the assay. The cells are then loaded with a fluorescence-imaging plate reader (FLIPR) calcium kit dye (Calcium 3 kit, Molecular Devices Ltd) containing 5 mM final concentration of probenecid and incubated at 37 °C for 1 hour in a 5 % CO₂ atmosphere. The fluorescence emission caused by intracellular calcium mobilization elicited by the PGD₂ at the CRTH2 receptor is determined with a FLEXstation benchtop scanning and integrated fluid transfer workstation (Molecular Devices Ltd). To detect antagonists and determine compound IC₅₀, compounds are pre-incubated at varying concentrations with the loaded cells for 15 minutes at 37 °C, 5 % CO₂ prior to the addition of the agonist at its EC₈₀ value. Compounds and agonist are added in Hanks balanced salt solution containing 20 mM HEPES and 0.1 % BSA). The fractional response values for each well are calculated by subtracting the basal response from the peak response. Results are calculated as the mean of triplicate wells using Excel and XLfit (Microsoft).

### Eosinophil Chemotaxis Assay

Eosinophils chemotaxis assay is performed immediately after isolating eosinophils from freshly collected blood. Whole citrated blood is spun in Accuspin tubes (Sigma, as are all other reagents) containing 15mL) histopaque at 800 g for 15 min (brake off). The granulocyte containing pellet is re-suspended in 20ml of 6% dextrane, than topped up to 50mL with PBS. The tubes are inverted 3-4 times and left to sediment for 45 minutes. The supernatant is collected and centrifuged at 800g for 10 minutes. The resulting pellet is re-suspended in 2mL PBS followed by 24mL sterile water. The remaining read blood cells are than lysed by adding 8mL 3.5 % NaCl and the suspension centrifuged at 800g for 10 minutes. The pellet is re-suspended in 1mL of running buffer (PBS with 0.5%BSA and 2mM EDTA) and the granulocytes counted in a haemocytometer. The cell suspension is centrifuged again at 800g for 10mins and the granulocyte pellet resuspended in 50µL buffer per 50 million cells. An equal volume of Miltenyi CD16 beads is added (Miltenyi Biotec, Germany) and the beads are incubated with cells for 30mins at 4°C. Wash the granulocyte/bead suspension with 1-2mL buffer per 10 million cells, spin down and re-suspend in 500µL buffer. Load the cell/bead suspension onto the Miltenyi column. Allow the cells to pass into the column and then elute the unlabelled (negative) fraction (eosinophils) with 30 mL buffer.

The chemotaxis assay is performed in disposable 96-well chambers (Neuoroprobe) The chemoattractant and control solutions are placed into appropriate wells of the lower section of the chamber (29□L, in PBS with 0.5% BSA and 2mM EDTA) and the framed membrane is fixed into place. 25µL of the cell suspension is pippetted onto the membrane above each of the filled lower .wells (200,000 cells per 25µL) and the chambers incubated for 2 hrs at 37°C. After the incubation, the remaining cells are aspirated from the top of membrane and the plate centrifuged at 2000rpm for 10min.
The membrane is than removed, the supernatants aspirated from each well and the plate frozen for at least 30mins at -80°C. Upon thawing 20µL of prepared Cyquant reagent is added to each well (Cyquant cell proliferation kit, Invitrogen). The fluorescence is measured using a plate reader with appropriate settings (Victor V, Perkin Elmer).

### Examples

The invention will now be described with reference to the following examples.

¹H NMR spectra were recorded at ambient temperature using a Varian Unity Inova (400MHz) spectrometer with a triple resonance 5 mm probe spectrometer. Chemical shifts are expressed in ppm relative to tetramethylsilane. The following abbreviations have been used: br s = broad singlet, s = singlet, d = doublet, dd = double doublet, t = triplet, q = quartet, m = multiplet.

Mass Spectrometry (LCMS) experiments to determine retention times and associated mass ions were performed using the following methods:
Method A: experiments were performed on a Micromass Platform LCT spectrometer with positive ion electrospray and single wavelength UV 254 nm detection using a Higgins Clipeus C18 5 µm 100 x 3.0 mm column and a 2 mL / minute flow rate. The initial solvent system was 95 % water containing 0.1 % formic acid (solvent A) and 5 % acetonitrile containing 0.1 % formic acid (solvent B) for the first minute followed by a gradient up to 5 % solvent A and 95 % solvent B over the next 14 minutes. The final solvent system was held constant for a further 2 minutes.
Method B: experiments were performed on a Micromass Platform LC spectrometer with positive and negative ion electrospray and ELS / Diode array detection using a Phenomenex Luna C18(2) 30 x 4.6 mm column and a 2 mL / minute flow rate. The solvent system was 95 % solvent A and 5 % solvent B for the first 0.50 minutes followed by a gradient up to 5 % solvent A and 95 % solvent B over the next 4 minutes. The final solvent system was held constant for a further 0.50 minutes

Microwave experiments were carried out using a Personal Chemistry Smith Synthesizer™, which uses a single-mode resonator and dynamic field tuning, both of which give reproducibility and control. Temperatures from 40-250 °C can be achieved, and pressures of up to 20 bar can be reached. Two types of vial are available for this processor, 0.5-2.0 mL and 2.0-5.0 mL.

Reverse-phase preparative HPLC purifications were carried out using Genesis 7 micron C-18 bonded silica stationary phase in columns 10 cm in length and 2 cm internal diameter. The mobile phase used was mixtures of acetonitrile and water (both buffered with 0.1 % v/v trifluoroacetic acid) with a flow rate of 10 mL per minute and typical gradients of 40 to 90 % organic modifier ramped up over 30 to 40 minutes. Fractions containing the required product (identified by LC-MS analysis) were pooled, the organic fraction removed by evaporation, and the remaining aqueous fraction lyophilised, to give the final product.

### Example 1: 3-(4-chlorophenylsulfanyl)-2-methylindolizin-1-yl]acetic acid

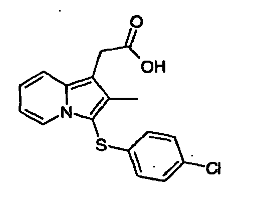

### Preparation (1a) (E)-3-pyridin-2-yl acrylic acid

A solution of pyridine-2-carbaldehyde (55 g), malonic acid (53 g), pyridine (90 mL) and piperidine (0.5 mL) was heated at 100 °C for 2 hours and then left at room temperature for 18 hours. The pH of the solution was adjusted to - 1 by the addition of 50 % aqueous hydrochloric acid and the resulting precipitate was collected by filtration, washed with cold water and dried to give title compound as a white solid, 21 g.
¹H NMR (DMSO-d6): δ 6.85 (d, J = 15.7 Hz, 1H), 7.40-7.45 (m, 1H), 7.60 (d, J = 15.7 Hz, 1H), 7.75 (m, 1H), 7.85-7.90 (m, 1H), 8.65 (m, 1H), 12.6 (br s, 1H).

### Preparation (1b) (E)-3-pyridin-2-yl acrylic acid methyl ester hydrochloride

To a suspension of (*E*)-3-pyridin-2-yl acrylic acid (21 g) in methanol (600 mL) at 0 °C was added thionyl chloride. The mixture was heated at reflux for one hour, cooled to room temperature, and the methanol removed under reduced pressure to afford a black semi-solid. Crystallisation from a mixture of ethanol and pentane gave title compound as an off-white solid, 15 g.
¹H NMR (CDCl₃): δ 3.85 (s, 3H), 7.55 (d, J = 16.2 Hz, 1H), 7.85-8.00 (m, 3H), 8.40 (m, 1H), 8.85 (m, 1H).

### Preparation (1c) 3-pyridin-2-yl propionic acid methyl ester hydrochloride

A mixture of (*E*)-3-pyridin-2-yl acrylic acid methyl ester hydrochloride (15 g) and 10 % w/w palladium on charcoal (0.75 g) in ethyl acetate (400 mL) was stirred under an atmosphere of hydrogen at room temperature for 2 days. The catalyst was removed by filtration and the filtrate concentrated under reduced pressure to afford title compound as a yellow oil, 15 g.
¹H NMR (CDCl₃): δ 3.15 (t, J = 7.1 Hz, 2H), 3.55 (t, J = 7.1 Hz, 2H), 3.65 (s, 3H), 7.75 (m, 1H), 7.80 (m, 1H), 8.30 (m, 1H), 8.65 (m, 1H).

### Preparation (1d) (2-methylindolizin-1-yl)acetic acid methyl ester

A mixture of 3-pyridin-2-yl propionic acid methyl ester hydrochloride (5.0 g), butan-2-one (100 mL), 1-chloropropan-2-one (2.9 mL) and sodium hydrogencarbonate (6.3 g) was heated at reflux for 4 days. The sodium hydrogencarbonate was removed by filtration and the filtrate concentrated under reduced pressure to afford a black oil. Purification of the residue by column chromatography on silica gel, eluting with a mixture of dichloromethane and pentane (1:9 to 9:1 by volume) gave title compound, 1.6 g.
¹H NMR (CDCl₃): δ 2.25 (s, 3H), 3.65 (s, 3H), 3.70 (s, 2H), 6.35-6.40 (m, 1H), 6.60-6.65 (m, 1H), 7.10(s, 1H), 7.30 (m, 1H), 7.75 (m, 1H).

Preparation (1e) [3-(4-chlorophenylsulfanyl)-2-methylindolizin-1-yl]acetic acid methyl ester

Bis(4-chlorophenyl)disulfide (0.070 g) and (2-methylindolizin-1-yl) acetic acid methyl ester (0.040 g) were dissolved in industrial methylated spirits and the resulting solution stirred at 80 °C for 3 days. The solvent was removed under reduced pressure and the residue purified by column chromatography on silica gel, eluting with a mixture of ethyl acetate and cyclohexane (1:19 by volume), to afford title compound as a green oil, 0.050 g.
¹H NMR (CDCl₃): δ 2.35 (s, 3H), 3.70 (s, 3H), 3.80 (s, 2H), 6.55 (m, 1H), 6.75-6.80 (m, 2H), 6.85-6.90 (m, 1H), 7.10-7.15 (m, 2H), 7.40 (m, 1H), 8.10 (m, 1H).

### Preparation (1f) [3-(4-chlorophenylsulfanyl)-2-methylindolizin-1-yl]acetic acid

0.15 M aqueous lithium hydroxide solution (5.0 mL) was added to a stirred solution of [3-(4-chlorophenylsulfanyl)-2-methylindolizin-1-yl] acetic acid methyl ester (0.050 g) in tetrahydrofuran (5.0 mL), and the resulting solution was heated at reflux for 18 hours. The pH of the solution was adjusted to ∼1 by the addition of dilute aqueous hydrochloric acid and the solvent removed under reduced pressure. The residue was dissolved in dichloromethane, washed with water and dried over magnesium sulfate and then concentrated under reduced pressure to afford title compound as an off-white solid, 0.035 g.
¹H NMR (DMSO-d6): δ 2.30 (s, 3H), 3.70 (s, 2H), 6.65 (m, 1H), 6.85 (m, 2H), 6.90 (m, 1H), 7.30 (m, 2H), 7.55 (m, 1H), 8.15 (m, 1H), 12.20 (br s, 1H).
MS: ESI (+ve) (Method A): 332 (M+H)⁺, Retention time 12.3 min.

### Example 2: [3-(4-chlorobenzenesulfonyl)-2-methylindolizin-1-yl]acetic acid

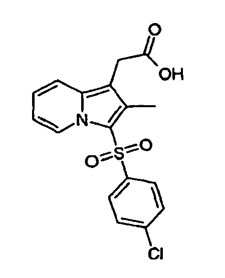

### Preparation (2a) [3-(4-chlorobenzenesulfonyl)-2-methylindolizin-1-yl]acetic acid

To a solution of [3-(4-chlorophenylsulfanyl)-2-methylindolizin-1-yl]acetic acid methyl ester (0.040 g) in methanol was added potassium carbonate (0.32 g) and 3-chloroperoxybenzoic acid (0.21 g) and the resulting mixture was stirred at room temperature for 18 hours. The methanol was removed under reduced pressure and the residue acidified by the addition of dilute aqueous hydrochloric acid. The mixture was extracted with ethyl acetate and the combined extracts dried over magnesium sulfate. The solvent was removed reduced pressure and the residue purified by column chromatography on silica gel, eluting with a mixture of ethyl acetate and cyclohexane (1:4 by volume), to afford title compound as an off-white solid, 0.032 g.
¹H NMR (CD₃OD): δ 2.60 (s, 3H), 3.75 (s, 2H), 6.80 (m, 1H), 7.05 (m, 1H), 7.50-7.55 (m, 3H), 7.85 (m, 2H), 8.85 (m, 1H).
MS: ESI (+ve) (Method A): 331 (M+H)⁺, Retention time 10.4 min.

### Example 3: [3-(4-methanesulfonylphenylsulfanyl)-2-methylindolizin-1-yl]acetic acid

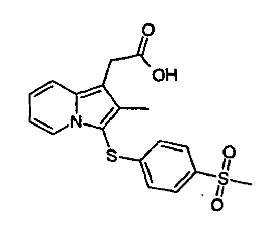

### Preparation (3a) [3-(4-methanesulfonylphenylsulfanyl)-2-methylindolizin-1-yl]acetic acid ethyl ester

Bis[4-(methylsulfonyl)phenyl]disulfide (0.22 g), (2-methylindolizin-1-yl)acetic acid methyl ester (0.087 g) and iodine (one crystal) were dissolved in ethanol and the resulting solution was heated at reflux for 16 hours. The solvent was removed under reduced pressure and the residue purified by column chromatography on silica gel, eluting with a mixture of dichloromethane and cyclohexane (1:1 to 19:1 by volume), to afford title compound, 0.049 g.
MS: ESI (+ve) (Method B): 404 (M+H)⁺, Retention time 3.85 min.

### Preparation (3b) [3-(4-methanesulfonylphenylsulfanyl)-2-methylindolizin-1-yl]acetic acid

To a solution of [3-(4-methanesuffonylphenylsulfanyl)-2-methylindolizin-1-yl]acetic acid ethyl ester (0.049 g) in methanol (0.75 mL) and water (0.25 mL) was added lithium hydroxide (0.020 g), and the resulting solution was stirred at room temperature for 2 hours. The solution was acidified by the addition of dilute aqueous hydrochloric acid and extracted with dichloromethane. The combined extracts were dried over magnesium sulfate and solvent removed under reduced pressure. Purification of the residue by column chromatography on silica gel, eluting with a mixture of acetone and cyclohexane, (1:10 to 3:7 by volume) afforded title compound.
¹H NMR (CDCl₃): 2.35 (s, 3H), 3.00 (s, 3H), 3.85 (s, 2H), 6.60 (m, 1H), 6.90-6.95 (m, 3H), 7.45 (m, 1H), 7.70 (m, 2H), 8.10 (m, 1H).
MS: ESI (+ve) (Method A): 376 (M+H)⁺, Retention time 9.9 min.

### Example 4: [3-(3,5-dichlorobenzenesulfonyl)-2-methylindolizin-1-yl]acetic acid

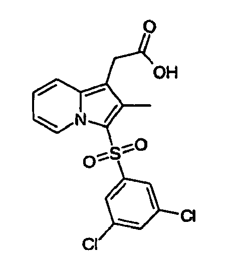

### Preparation (4a) [3-(3,6-dichlorobenzenesulfonyl)-2-methylindolizin-1-yl]acetic acid methyl ester

Bis(3,5-dichlorophenyl)disulfide (0.24 g) and (2-methylindolizin-1-yl)acetic acid methyl ester (0.066 g) were dissolved in industrial methylated spirits (6.0 mL) and the resulting solution stirred at 80 °C for 3 days. The solvent was removed under reduced pressure and purification of the residue by column chromatography on silica gel, eluting with a mixture of ethyl acetate and cyclohexane (1:19 by volume), gave title compound, 0.096 g.
¹H NMR (CD₃OD): δ 2.25 (s, 3H), 3.60 (s, 3H), 3.90 (s, 2H), 6.75 (m, 1H), 6.80 (d, J = 1.8 Hz, 2H), 6.9-7.00 (m, 1H), 7.40 (t, J = 1.8 Hz, 1H), 7.60 (m, 1H), 8.20 (m, 1H).

### Preparation (4b) [3-(3,5-dichlorobenzenesulfonyl)-2-methylindolizin-1-yl]acetic acid

To a solution of [3-(3,5-dichlorobenzenesulfonyl)-2-methylindolizin-1-yl]acetic acid methyl ester (0.096 g) in methanol (15 mL) was added potassium carbonate (1.3 g) and 3-chloroperoxybenzoic acid (0.65 g) and the resulting solution was stirred at room temperature for 2 days. The methanol was removed under reduced pressure and the residue acidified by the addition of dilute aqueous hydrochloric acid. The mixture was extracted with ethyl acetate and the combined extracts dried over magnesium sulfate. The solvent was removed under reduced pressure and the residue purified by preparative reverse-phase HPLC using a gradient over 55 minutes of acetonitrile in water (20 % to 80 % of organic modifier) to afford title compound, 0.016 g.
¹H NMR (CD₃OD): δ 2.55 (s, 3H), 3.75 (s, 2H), 6.90 (m, 1H), 7.15 (m, 1H), 7.55 (m, 1H) 7.70 (t, J =1.9 Hz, 1H), 7.80 (d, J = 1.9 Hz, 2H), 8.90 (m, 1H).
MS: ESI (+ve) (Method A): 397 (M+H)⁺, Retention time 11.3 min.

### Example 5: 3-(4-chlorophenylmethanesulfonyl)-2-methylindolizin-1-yl]acetic acid

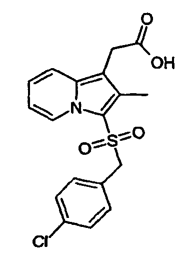

### Preparation (5a) 3-(4-chlorophenylmethanesulfonyl)-2-methylindolizin-1-yl]acetic acid methyl ester

Bis(4-chlorobenzyl)disulfide (0.21 g), (2-methylindolizin-1-yl)acetic acid methyl ester (0.066 g) and iodine (one crystal) were dissolved in industrial methylated spirits (6.0 mL) and the resulting solution stirred at 80 °C for 3 days. Purification of the reaction mixture by column chromatography on silica gel, eluting with a mixture of ethyl acetate and cyclohexane (1:19 by volume), gave title compound, 0.049 g.
¹H NMR (CD₃OD): δ 1.90 (s, 3H), 3.60 (s, 3H), 3.70 (s, 2H), 3.80 (s, 2H), 6.60 (m, 1H), 6.80-6.85 (m, 1H), 6.90 (m, 2H), 7.20 (m, 2H), 7.40 (m, 1H), 8.25 (m, 1H).

### Preparation (5b) 3-(4-chlorophenylmethanesulfonyl)-2-methylindolizin-1-yl]acetic acid

To a solution of [3-(4-chlorophenylmethanesulfonyl)-2-methylindolizin-1-yl]acetic acid methyl ester (0.049 g) in methanol (10 mL) was added potassium carbonate (0.44 g) and 3-chloroperoxybenzoic acid (0.44 g) and the resulting solution was stirred at room temperature for 18 hours. The solvent was removed under reduced pressure and purification of the residue by preparative reverse-phase HPLC using a gradient over 55 minutes of acetonitrile in water (20 % to 80 % of organic modifier) gave title compound, 0.020 g
¹H NMR (CD₃OD): δ 2.25 (s, 3H), 3.70 (s, 2H), 4.45 (s, 2H), 6.55 (m, 1H), 6.85 (m, 2H), 7.00-7.05 (m, 1H), 7.10 (m, 2H), 7.50 (m, 1H), 8.45 (m 1H).
MS: ESI (+ve) (Method A): 377 (M+H)⁺, Retention time 10.0 min.

### Reference Example 6: [2-methyl-3-(naphthalene-2-sulfonyl)indolizin-1-yl]acetic acid

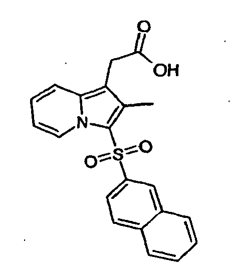

### Preparation 6a: bis(2-naphthyl)disulfide

Bromine (1.2 g) was added dropwise to a mixture of naphthalene-2-thiol (2.0 g), ethanol (6.0 mL) and ethyl acetate (6.0 mL) at room temperature and the resulting mixture was stirred at room temperature for 18 hour. The mixture was diluted with ethyl acetate, washed saturated aqueous sodium thiosulfate solution and dried over magnesium sulfate. The solvent was removed under reduced pressure to afford title compound, 2.0 g
¹H NMR (CDCl₃): δ 7.40-7.50 (m, 4H), 7.60 (dd, J = 1.8, 8.7 Hz, 2H), 7.75 (m, 2H), 7.80 (m, 4H), 8.00 (d, J =1.8 Hz, 2H).

### Preparation 6: [2-methyl-3-(naphthalene-2-sulfonyl)indolizin-1-yl]acetic acid ethyl ester

Bis(2-naphthyl)disulfide (0.47 g), (2-methylindolizin-1-yl)acetic acid methyl ester (0.15 g) and iodine (1 crystal) were dissolved in industrial methylated spirits (15 mL) and the resulting solution stirred at 80 °C for 24 hours. The solvent was removed under reduced pressure and purification of the residue by column chromatography on silica gel, eluting with a mixture of ethyl acetate and cyclohexane (1:19 by volume), gave title compound, 0.12 g.
MS: ESI (+ve) (Method B): 376 (M+H)⁺, Retention time 4.7 min.

### Preparation 6: [2-methyl-3-(naphthalene-2-sulfonyl)indolizin-1-yl]acetic acid

To a solution of [2-methyl-3-(naphthalene-2-sulfonyl)indolizin-1-yl]acetic acid ethyl ester (0.12 g) in methanol (20 mL) was added potassium carbonate (0.46 g) and 3-chloroperoxybenzoic acid (0.30 g) and the resulting solution was stirred at room temperature for 18 hours. The methanol was removed under reduced pressure and purification of the residue by preparative reverse-phase HPLC using a gradient over 40 minutes of acetonitrile in water gave title compound as a blue solid, 0.0032 g.
¹H NMR (CD₃OD): δ 2.65 (s, 3H), 3.70 (s, 2H), 6.80 (dt, J = 1.1, 6.8 Hz, 1H), 7.05 (dd, J = 0.9, 6.8 Hz, 1H), 7.50 (m, 1H), 7.60 (m, 2H), 7.75 (dd, J = 1.8, 8.8 Hz, 1H), 7.90 (m, 1H), 7.95 (dd, J = 8.8 Hz, 1H), 8.05 (m, 1H), 8.55 (d, J = 1.5 Hz, 1H), 8.95 (m, 1H).
MS: ESI (+ve) (Method A): 380 (M+H)⁺, Retention time 10.6 min.
MS: ESI (+ve) (Method B): 380 (M+H)⁺, Retention time 3.5 min.

### Reference Example 7: [2-methyl-3-(naphthalen-1-ylsulfanyl)indolizin-1-yl]acetic acid

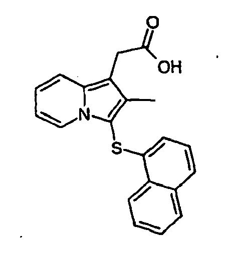

### Preparation 7a: bis(1-naphthyl)disulfide

Bromine (1.2 g) was added dropwise to a mixture of naphthalene-1-thiol (2.0 g), ethanol (6.0 mL) and ethyl acetate (6.0 mL) at room temperature and the resulting mixture was stirred at room temperature for 18 hour. The mixture was diluted with ethyl acetate, washed with saturated aqueous sodium thiosulfate solution and dried over magnesium sulfate. The solvent was removed under reduced pressure and the residue triturated with ethanol to afford title compound, 2.2 g.
¹H NMR (CDCl₃): δ 7.30 (dd, J = 7.2, 8.1 Hz, 2H), 7.50-7.55 (m, 4H), 7.60 (dd, J = 1.2, 7.2 Hz, 2H), 7.80 (m, 2H), 7.85 (m, 2H), 8.35 (m, 2H).

### Preparation 7b: [2-methyl-3-(naphthalen-1-ylsulfanyl)indolizin-1-yl]acetic acid ethyl ester

Bis(1-naphthyl)disulfide (0.47 g), (2-methylindolizin-1-yl)acetic acid methyl ester (0.15 g) and iodine (1 crystal) were dissolved in industrial methylated spirits (15 mL) and the resulting solution stirred at 80 °C for 24 hours. The solvent was removed under reduced pressure and purification of the residue by column chromatography on silica gel, eluting with a mixture of ethyl acetate and cyclohexane (1:19 by volume), gave title compound, 0.13 g.
MS: ESI (+ve) (Method B): 376 (M+H)⁺, Retention time 4.7 min.

### Preparation 7c: [2-methyl-3-(naphthalen-1-ylsulfanyl)indolizin-1-yl]acetic acid

To a solution of [2-methyl-3-(naphthalen-1-ylsulfanyl)indolizin-1-yl]acetic acid ethyl ester (0.13 g) in methanol (20 mL) was added potassium carbonate (0.50 g), and then 3-chloroperoxybenzoic acid (0.32 g) and the resulting mixture was stirred at room temperature for 48 hours. The methanol was removed under reduced pressure and the residue acidified by the addition of dilute aqueous hydrochloric acid. The mixture was extracted with ethyl acetate and the combined extracts dried over magnesium sulfate. The solvent was removed under reduced pressure and purification of the residue by preparative reverse-phase HPLC using a gradient over 40 minutes of acetonitrile in water to afford title compound, 0.010 g.
¹H NMR (CD₃OD): δ 2.40 (s, 3H), 3.80 (s, 2H), 6.30 (dd, J = 0.9, 7.3 Hz, 1H), 6.55 (m, 1H), 6.85 (m, 1H), 7.10 (t, J = 7.7 Hz, 1H), 7.45-7.65 (m, 4H), 7.85 (d, J = 7.7 Hz, 1H), 8.05 (m, 1H), 8.40 (m, 1H).
MS: ESI (+ve) (Method A): 348 (M+H)⁺, Retention time 12.9 min.
MS: ESI (+ve) (Method B): 348 (M+H)⁺, Retention time 4.2 min.

### Reference Example 8: [2-methyl-3-(quinolin-8-ylsulfanyl)indolizin-1-yl]acetic acid

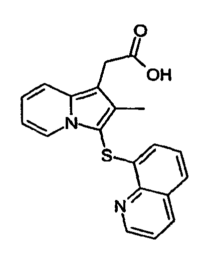

### Preparation 8a: [2-methyl-3-(quinolin-8-ylsulfanyl)indolizin-1-yl]acetic acid methyl ester

Bis(8-quinolyl)disulfide (0.36 g), (2-methylindolizin-1-yl)acetic acid methyl ester (0.15 g) and iodine (1 crystal) were dissolved in industrial methylated spirits (15 mL) and the resulting solution stirred at 80 °C for 24 hours. The solvent was removed under reduced pressure and purification of the residue by column chromatography on silica gel, eluting with a mixture of ethyl acetate, cyclohexane and triethylamine (1:19:0 to 8.5:1:0.5 by volume) gave title compound as a yellow oil, 0.20 g.
¹H NMR (DMSO-d6): δ 2.35 (s, 3H), 3.70 (s, 3H), 3.90 (s, 2H), 6.35 (dd, J = 1.3, 7.4 Hz, 1H), 6.55 (dt, J = 1.1, 6.6 Hz, 1H), 6.85 (m, 1H), 7.25 (t, J = 8.0 Hz, 1H), 7.50 (m, 1H), 7.55 (dd, J = 4.2, 8.2 Hz, 1H), 7.60 (dd, J = 1.0, 8.2 Hz, 1H), 8.10 (m, 1H), 8.30 (dd, J = 1.7, 10.0 Hz, 1H), 8.90 (dd, J = 1.7, 4.2 Hz, 1H).
MS: ESI (+ve) (Method B): 363 (M+H)⁺, Retention time 4.1 min.

### Preparation 8b: [2-methyl-3-(quinolin-8-ylsulfanyl)indolizin-1-yl]acetic acid

To a solution of [2-methyl-3-(quinolin-8-ylsulfanyl)indolizin-1-yl]acetic acid methyl ester (0.20 g) in methanol (30 mL) was added potassium carbonate (1.5 g) and 3-chloroperoxybenzoic acid (1.0 g) and the resulting solution was stirred at room temperature for 48 hours. The methanol was removed under reduced pressure and the residue purified by preparative reverse-phase HPLC using a gradient over 40 minutes of acetonitrile in water to afford title compound as a yellow solid, 0.020 g.
¹H NMR (CD₃OD): δ 2.35 (s, 3H), 3.85 (s, 2H), 6.40 (dd, J = 1.2, 7.4 Hz, 1H), 6.55 (m, 1H), 6.85 (m, 1H), 7.25 (t, J = 7.9 Hz, 1H), 7.50 (dt, J = 1.2, 8.9 Hz, 1H), 7.60 (dd, J = 4.3, 8.3 Hz, 1H), 7.65 (dd, J = 1.2, 8.3 Hz, 1H), 8.10 (dt, J = 1.2, 7.0 Hz, 1H), 8.35 (dd, J = 1.6, 8.3 Hz, 1H), 8.90 (dd, J = 1.6, 4.2 Hz, 1H).
MS: ESI (+ve) (Method A): 349 (M+H)⁺, Retention time 10.5 min.

### Example 9: [7-chloro-3-(4-methanesulfonylphenylsulfanyl)-2-methylindolizin-1-yl]acetic acid

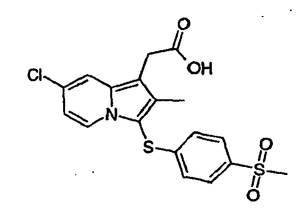

### Preparation 9a: 7-chloro-2-methylindolizine

A mixture of 1-chloropropan-2-one (0.85 mL), lithium bromide (0.89 g) and acetonitrile (15 mL) was stirred at room temperature for 15 minutes and then treated with a solution of 4-chloro-2-methylpyridine (1.0 g) in acetonitrile (15 mL) and the resulting mixture was heated at reflux for 24 hours. The mixture was cooled to room temperature and the solvent removed under reduced pressure. The residue was diluted with water, extracted with diethyl ether and the aqueous phase was treated with potassium carbonate (1.6 g) and then heated at 80 °C for 2 hours. The mixture was cooled to room temperature, extracted with ethyl acetate and the combined extracts dried over magnesium sulfate. The solvent was removed under reduced pressure to afford title compound, 0.71 g.
¹H NMR (CDCl₃): δ 2.30 (s, 3H), 6.20 (s, 1H), 6.35 (dd, J = 2.1, 7.3 Hz, 1H), 7.05 (m, 1H), 7.20 (dd, J = 2.1 Hz, 1H), 7.70 (dt, J = 0.8, 7.3 Hz, 1H).
MS: ESI (+ve) (Method B): 166 (M+H)⁺, Retention time 3.9 min.

### Preparation 9b: 7-chloro-3-(4-methanesulfonylphenylsulfanyl)-2-methylindolizine

Bis[4-(methylsulfonyl)phenyl]disulfide (1.9 g) and 7-chloro-2-methylindolizine (0.71 g) were dissolved in ethanol and the resulting solution was heated at 80 °C for 18 hours. The solvent was removed under reduced pressure and purification of the residue by column chromatography on silica gel gave title compound, 0.26 g.
¹H NMR (CDCl₃): δ 2.35 (s, 3H), 3.00 (s, 3H), 6.45 (s, 1H), 6.55 (dd, J = 2.1, 7.5 Hz, 1H), 6.95 (d, J = 8.7 Hz, 2H), 7.35 (dd, J = 0.7, 2.1 Hz, 1H), 7.55 (d, J = 8.7 Hz, 2H), 8.00 (dt, J = 0.7, 7.3 Hz, 1H).

### Preparation 9c: [7-chloro-3-(4-methanesulfonylphenylsulfanyl)-2-methylindolizin-1-yl]oxoacetic acid methyl ester

A solution of 7-chloro-3-(4-methanesulfonylphenylsulfanyl)-2-methylindolizine (0.24 g) and tetrahydrofuran (5.0 mL) at room temperature was treated with chlorooxoacetic acid methyl ester (0.1 g) and the resulting solution stirred at room temperature for 18 hours. The solvent was removed under reduced pressure and purification of the residue by column chromatography on silica gel, eluting with a mixture of cyclohexane and ethyl acetate (9:1 to 4:1 to 7:3 by volume), gave title compound as a yellow oil, 0.27 g.
¹H NMR (CDCl₃): δ 2.50 (s, 3H), 3.00 (s, 3H), 4.00 (s, 3H), 7.00 (dd, J =2.2, 7.3 Hz, 1H), 7.05 (d, J = 8.7 Hz, 2H), 7.80 (d, J = 8.7 Hz, 2H), 8.25 (dd, J = 0.7, 7.3 Hz, 1H), 8.50 (dd, J =0.7, 2.2 Hz, 1H).
MS: ESI (+ve) (Method B): 438 (M+H)⁺, Retention time 3.8 min.

### Preparation 9d: [7-chloro-3-(4-methanesulfonylphenylsulfanyl)-2-methylindolizin-1-yl]hydroxyacetic acid methyl ester

A solution of sodium borohydride (0.11 g) in methanol (14 mL) and water (0.5 mL) at -40 °C was treated with a solution of [7-chloro-3-(4-methanesulfonylphenylsulfanyl)-2-methylindolizin-1-yl]oxoacetic acid methyl ester (0.27 g) in methanol (1.0 mL) and dichloromethane (1.0 mL) and the resulting solution was stirred at -40 °C for 1 hour. The solution was diluted with 10 % aqueous acetic acid (10 mL), extracted with dichloromethane and the combined extracts dried over magnesium sulfate. The solvent was removed under reduced pressure and the residue triturated with water to afford title compound as a green solid, 0.10g.
¹H NMR (DMSO-d6): δ 2.30 (s, 3H), 3.15 (s, 3H), 3.60 (s, 3H), 5.55 (s, 1H), 6.75 (dd, J = 2.2, 7.5 Hz, 1H), 7.00 (d, J = 8.7 Hz, 2H), 7.80 (d, J = 8.7 Hz, 2H), 7.85 (dd, J = 0.7, 2.2 Hz, 1H), 8.20 (dd, J = 0.7, 7.5 Hz, 1H).

### Preparation 9e: (7-chloro-3-(4-methanesulfonylphenylsulfanyl)-2-methylindolizin-1-yl]acetic acid methyl ester

A mixture of triphenylphosphine (0.060 g), iodine (0.029 g) and toluene (1.5 mL) was stirred at room temperature for 20 minutes and then treated with a solution of [7-chloro-3-(4-methanesulfonylphenylsulfanyl)-2-methylindolizin-1-yl]hydroxyacetic acid methyl ester (0.050 g) in toluene (5.0 mL) and dichloromethane (5.0 mL) and the resulting mixture was heated at 80 °C for 4 hours. The mixture was cooled to room temperature and the solvent removed under reduced pressure. The residue was dissolved in dichloromethane, washed with saturated aqueous sodium thiosulfate solution and dried over magnesium sulfate. The solvent was removed under reduced pressure and purification of the residue by column chromatography on silica gel, eluting with a mixture of cyclohexane and ethyl acetate (4:1 by volume) gave title compound, 0.018 g.
¹H NMR (CDCl₃): δ 2.35 (s, 3H), 3.00 (s, 3H), 3.70 (s, 3H), 3.75 (s, 2H), 6.55 (dd, J = 2.1, 7.4 Hz, 1H), 6.95 (d, J = 8.7 Hz, 2H), 7.45 (dd, J = 0.8, 2.1 Hz, 1H), 7.70 (d, J = 8.7 Hz, 2H), 8.00 (J = 0.8, 7.4 Hz, 1H).
MS: ESI (+ve) (Method B): 424 (M+H)⁺, Retention time 4.0 min.

### Preparation 9f: [7-chloro-3-(4-methanesulfonylphenylsulfanyl)-2-methylindolizin-1-yl]acetic acid

A mixture of [7-chloro-3-(4-methanesulfonylphenylsulfanyl)-2-methylindolizin-1-yl]acetic acid methyl ester (0.018 g), tetrahydrofuran (2.0 mL), water (2.0 mL) and lithium hydroxide (0.0090 g) was stirred at room temperature for 2 hours. The solvent was removed under reduced pressure and the residue acidified by the addition of 1.0 M aqueous hydrochloric acid and then extracted with ethyl acetate. The combined extracts were dried over magnesium sulfate and solvent removed under reduced pressure. The residue was purified by column chromatography on silica gel, followed by trituration with pentane and diethyl ether to afford title compound as a yellow solid, 0.0022 g.
¹H NMR (CD₃OD): δ 2.35 (s, 3H), 3.05 (s, 3H), 3.75 (s, 2H), 6.60 (dd, J = 2.2, 7.5 Hz, 1H), 7.05. (d, J = 8.6 Hz, 2H), 7.55 (m 1H), 7.75 (d, J = 8.6 Hz, 2H), 8.10 (d, J = 7.5 Hz, 1H).
MS: ESI (+ve) (Method A): 410 (M+H)⁺, Retention time 10.4 min.
MS: ESI (+ve) (Method B): 410 (M+H)⁺, Retention time 3.5 min.

### Examples 10: [6-fluoro-3-(4-methanesulfonylphenylsulfanyl)-2-methylindolizin-1-yl]acetic acid

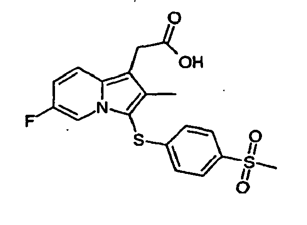

### Preparation 10a: (E)-3-(5-fluoropyridin-2-yl)acrylic acid hydrochloride

A mixture of 5-fluoropyridine-2-carbaldehyde (10 g), malonic acid (8.3 g), pyridine (20 mL) and piperidine (3-4 drops) was heated at 100 °C for 2 hours and then left at room temperature for 18 hours. The mixture was cooled to 0 °C and acidified by the addition of 50 % aqueous hydrochloric acid. The resulting precipitate was collected by filtration, washed with water and diethyl ether and then dried to afford title compound as a white solid, 10g.
¹H NMR (DMSO-d6): δ 6.75 (dd, J = 0.6, 15.8 Hz, 1H), 7.60 (d, J = 15.8 Hz, 1H), 7.75-7.85 (m, 2H), 8.65 (d, J = 2.7 Hz, 1H), 12.60 (br s, 1H).

### Preparation 10b: (E)-3-(5-fluoropyridin-2-yl)acrylic acid methyl ester

A mixture of (*E*)-3-(5-fluoropyridin-2-yl)acrylic acid hydrochloride (10 g), sulfuric acid (2.0 mL) and methanol was stirred at room temperature for 17 hours and then at 50 °C for 4 hours. The mixture was cooled to 0 °C and the pH of the solution adjusted to 8-9 by the addition of 1.0 M aqueous sodium hydroxide solution. The resulting precipitate was collected by filtration, washed with water and dried to afford title compound as a white solid, 9.0 g.
¹H NMR (DMSO-d6): δ 3.75 (s, 3H), 6.85 (dd, J = 0.6, 15.7 Hz, 1H), 7.70 (d, J = 15.7 Hz, 1H), 7.80-7.90 (m, 2H), 8.65 (d, J = 2.9 Hz, 1H).
MS: ESI (+ve) (Method B): 182 (M+H)⁺, Retention time 2.8 min.

### Preparation 10c: 3-(5-fuoropyridin-2-yl)propionic acid methyl ester

A mixture of (*E*)-3-(5-fluoropyridin-2-yl)acrylic acid methyl ester (8.0 g), 10 % w/w palladium on charcoal (0.50 g) and ethyl acetate (60 mL) was stirred under an atmosphere of hydrogen at room temperature for 18 hours. The catalyst was removed by filtration and the filtrate concentrated under reduced pressure to afford title compound as a yellow oil, 6.0 g.
¹H NMR (CDCl₃): δ 2.80 (t, J = 7.3 Hz, 2H), 3.10 (t, J = 7.3 Hz, 2H), 3.65 (s, 3H), 7.20 (dd, J = 4.4, 8.5 Hz, 1H), 7.30 (dt, J = 2.9, 8.5 Hz, 1H), 8.35(d, J = 2.9 Hz, 1H).

### Preparation 10d: (6-fluoro-2-methylindolizin-1-yl)acetic acid

A mixture of 1-chloropropan-2-one (6.0 mL), lithium bromide (5.3 g) and acetonitrile (75 mL) was stirred at room temperature for 15 minutes and then treated with a solution of 3-(5-fuoropyridin-2-yl)propionic acid methyl ester (7.0 g) in acetonitrile (75 mL) and the resulting mixture was heated at reflux for 24 hours. The mixture was cooled to room temperature and the solvent removed under reduced pressure. The residue was diluted with water, extracted with diethyl ether and the aqueous phase was treated with potassium carbonate (7.9 g) and then heated at 80 °C for 1 hour. The mixture was cooled to room temperature, acidified by the addition of 1.0 M aqueous hydrochloric acid and extracted with ethyl acetate. The combined extracts were dried over magnesium sulfate and the solvent removed under reduced pressure to afford title compound as a dark green solid, 1.4 g.
MS: ESI (+ve) (Method B): 208 (M+H)⁺, Retention time 2.8 min.

### Preparation 10e: (6-fluoro-2-methylindolizin-1-yl)acetic acid methyl ester

A mixture of (6-fluoro-2-methylindolizin-1-yl)acetic acid (1.4 g), 1,3-dicyclohexylcarbodiimide (1.5 g), 4-(dimethylamino)pyridine (0.082 g), methanol (0.81 mL) and dichloromethane (50 mL) was stirred a room temperature for 3 days. The solvent was removed under reduced pressure and the residue purified by column chromatography on silica gel, eluting with dichloromethane to afford title compound as a yellow oil, 0.65 g.
¹H NMR (CDCl₃): δ 2.25 (s, 3H), 3.65 (s, 3H), 3.70 (s, 2H), 6.55 (m, 1H), 7.10 (m, 1H), 7.25 (dd, J = 5.8, 9.8 Hz, 1H), 7.70 (m, 1H).

### Preparation 10f: [6-fluoro-3-(4-methanesulfonylphenylsulfanyl)-2-methylindolizin-1-yl]acetic acid methyl ester

A mixture of (6-fluoro-2-methylindolizin-1-yl)acetic acid methyl ester (0.65 g), bis[4-(methylsulfonyl)phenyl]disulfide (2.2 g) and methanol were heated a reflux for 24 hours. The mixture was cooled to room temperature and the solvent removed under reduced pressure. The residue was purified by column chromatography on silica gel, eluting with a mixture of dichloromethane and ethyl acetate (1:0 to 1:1 by volume), to afforded title compound as a pale green oil, 0.18 g.
MS: ESI (+ve) (Method B): 408 (M+H)⁺, Retention time 3.9 min.

### Preparation 10g: [6-fluoro-3-(4-methanesulfonylphenylsulfanyl)-2-methylindolizin-1-yl]acetic acid

A mixture of [6-fluoro-3-(4-methanesulfonylphenylsulfanyl)-2-methylindolizin-1-yl]acetic acid methyl ester (0.16 g), methanol (10 mL) and 5.0 M aqueous sodium hydroxide solution (1.0 mL) was stirred at room temperature for 18 hours. The mixture was acidified by the addition of glacial acetic acid and the solvent removed under reduced pressure. The residue was purified by preparative reverse-phase HPLC using a gradient over 30 minutes of acetonitrile in water (35 % to 95 % of organic modifier) to afford title compound as a pale green solid, 0.050 g.
¹H NMR (DMSO-d6): δ 2.25 (s, 3H), 3.15 (s, 3H), 3.80 (s, 2H), 7.00 (m, 3H), 7.65 (dd, J = 5.7, 9.7 Hz, 1H), 7.75 (d, J = 8.6 Hz, 2H), 8.25 (dd, J = 2.1, 5.3 Hz, 1H), 12.30 (s, 1H).
MS: ESI (+ve) (Method A): 394 (M+H)⁺, Retention time 9.8 min.
MS: ESI (+ve) (Method B): 394 (M+H)⁺, Retention time 3.5 min.

### Example 11: [3-(4-chlorophenylsulfanyl)-2-methyl-7-trifluoromethylindolizin-1-yl]acetic acid

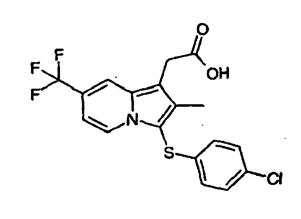

### Preparation 11a: 3-(4-trifluoromethylpyridin-2-yl)propionic acid ethyl ester

A solution of 3-ethoxy-3-oxopropylzinc bromide in tetrahydrofuran (0.5 M, 67 mL) was added dropwise over a period of 1 hour to a mixture of 2-bromo-4-trifluoromethylpyridine (6.9 g), tetrakis(triphenylphosphine)palladium(0), (0.60 g) and tetrahydrofuran (20 mL) at room temperature. The resulting mixture was stirred at room temperature for 18 hours, diluted with water (150 mL) and extracted with methyl *tert*-butyl ether. The combined extracts were dried over magnesium sulphate and the solvent removed under reduced pressure. Purification of the residue by column chromatography on silica gel, eluting with a mixture of pentane, dichloromethane and ethyl acetate (1:1:0, 0:1:0 and 0:5:1 by volume), gave title compound as a yellow oil, 4.2 g.
¹H NMR (CDCl₃): δ 1.25 (t, J = 7.0 Hz, 3H), 2.85 (t, J = 7.3 Hz, 2H), 3.20 (t, J = 7.3 Hz, 2H), 4.10 (q, J = 7.0 Hz, 2H), 7.35 (d, J = 4.7 Hz, 1H), 7.40 (s, 1H), 8.70 (d, J = 4.7 Hz, 1H).
MS: ESI (+ve) (Method B): 248 (M+H)⁺, Retention time 3.33 min.

### Preparation 11b: (2-methyl-7 -trifluoromethylindolizin-1-yl)acetic acid ethyl ester

A mixture of 1-chloropropan-2-one (2.0 mL), lithium bromide (2.2 g) and acetonitrile (40 mL) was heated at reflux for 15 minutes and then treated with a solution of 3-(4-trifluoromethylpyridin-2-yl)propionic acid ethyl ester (4.1 g) in acetonitrile (35 mL) and the resulting mixture was heated at reflux for 24 hours. The mixture was cooled to room temperature and the solvent removed under reduced pressure. The residue was diluted with water and the pH adjusted to 4 by the addition sodium acetate and then extracted with ethyl acetate. The combined extracts were dried over magnesium sulfate and the solvent removed under reduced pressure. The residue was purified by column chromatography on silica gel, eluting with a mixture of pentane and dichloromethane (4:1 to 1:4 by volume) to afford title compound as a yellow oil, 0.18 g.
MS: ESI (+ve) (Method B): 286 (M+H)⁺, Retention time 4.0 min.

### Preparation 11c: [3-(4-chlorophenylsulfanyl)-2-methyl-7-trifluoromethylindolizin-1-yl]acetic acid ethyl ester

Bis(4-chlorophenyl)disulfide (0.13 g) was added to a solution of sulfuryl chloride (0.031 mL) in 1,2-dichloroethane (2.5 mL) at 0 °C and the resulting solution was stirred at room temperature for 1 hour. This solution was added to a solution of (2-methyl-7-trifluoromethylindolizin-1-yl)acetic acid ethyl ester (0.17 g) in *N,N-*dimethylformamide (2.5 mL) at room temperature and the resulting solution was stirred at room temperature for 24 hours. The mixture was diluted with saturated aqueous sodium hydrogen carbonate solution (5.0 mL) and extracted with ethyl acetate. The combined extracts were washed with saturated aqueous sodium chloride solution, dried over magnesium sulfate and the solvent removed under reduced pressure. Purification of the residue by column chromatography on silica gel, eluting with a mixture of pentane and dichloromethane (4:1 to 0:1 by volume), gave title compound as a pale yellow solid, 0.13 g.

MS: ESI (+ve) (Method B): 248 (M+H)⁺, Retention time 3.33 min.

### Preparation 11d: (3-(4-chlorophenylsulfanyl)-2-methyl-7-trifluoromethylindolizin-1-yl]acetic acid

A mixture of [3-(4-chlorophenylsulfanyl)-2-methyl-7-trifluoromethylindolizin-1-yl]acetic acid ethyl ester (0.13 g), methanol (4.0 mL), water (0.4 mL) and 5.0 M aqueous lithium hydroxide solution (0.2 mL) was stirred at room temperature for 1.5 hours and then at 40 °C for 2 hours. The mixture was acidified by the addition of glacial acetic acid and the solvent removed under reduced pressure. The residue was diluted with water and extracted with ethyl acetate. The combined extracts were dried over magnesium sulfate and the solvent removed under reduced pressure. The residue was triturated with a mixture of acetonitrile (1.0 mL), water (2.0 mL) and *N*,*N-*dimethylformamide (1.0 mL) to afford title compound as a pale blue solid, 0.070 g.
¹H NMR (DMSO-d6): δ 2.30 (s, 3H), 3.90 (s, 2H), 6.90 (m, 3H), 7.35 (d, J = 8.6 Hz, 2H), 8.10 (s, 1H), 8.30 (d, J = 7.3 Hz, 1H), 12.35 (br s, 1H).
MS: ESI (+ve) (Method A): 400 (M+H)⁺, Retention time 13.0 min.
MS: ESI (+ve) (Method B): 400 (M+H)⁺, Retention time 4.4 min.

### Example 12: [3-(4-methanesulfonylphenylsulfanyl)-2-methyl-7-trifluoromethylindolizin-1-yl]acetic acid

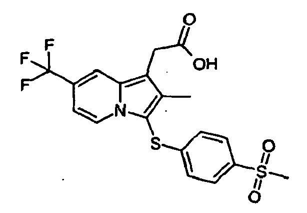

### Preparation 12a: [3-(4-methanesulfonylphenylsulfanyl)-2-methyl-7-trifluoromethylindolizin-1-yl]acetic acid ethyl ester

Sulfuryl chloride (0.057 mL) was added to a solution of bis(4-methylsulfonylphenyl) disulfide (0.32 g) in dichloromethane ( 10 mL) at 0 °C and the resulting solution was stirred at room temperature for 1 hour. This solution was added to a solution of (2-methyl-7-trifluoromethylindolizin-1-yl)acetic acid ethyl ester (0.20 g) in *N*,*N-*dimethylformamide (1.0 mL) at room temperature and the resulting solution was stirred at room temperature for 1 hour. The mixture was washed with saturated aqueous sodium hydrogen carbonate solution (5.0 mL) and saturated aqueous sodium chloride solution, dried over magnesium sulfate and the solvent removed under reduced pressure. The residue was purified by column chromatography on silica gel, eluting with a mixture of pentane and dichloromethane (1:1 to 0:1 by volume), to afforded title compound as a cream solid, 0.20 g.
¹H NMR (CDCl₃): δ 1.25 (t, J = 7.1 Hz, 3H), 2.40 (s, 3H), 3.00 (s, 3H), 3.80 (s, 2H), 4.20 (q, J = 7.1 Hz, 2H), 6.70 (dd, J = 1.8, 7.4 Hz, 1H), 6.95 (d, J = 8.7 Hz, 2H), 7.75 (d, J = 8.7 Hz, 2H), 7.80 (m, 1H), 8.15 (d, J = 7.4 Hz, 1H).
MS: ESI (+ve) (Method B): 472 (M+H)⁺, Retention time 4.3 min.

Preparation 12b: [3-(4-methanesulfonylphenylsulfanyl)-2-methyl-7-trifluoromethylindolizin-1-yl]acetic acid

A mixture of [3-(4-methanesulfonylphenylsulfanyl)-2-methyl-7-trifluoromethylindolizin-1-yl]acetic acid ethyl ester (0.19 g), ethanol (8.0 mL), water (1.0 mL) and 5.0 M aqueous lithium hydroxide solution (0.5 mL) was stirred at 50 °C for 2 hours. The mixture was cooled to room temperature, acidified by the addition of glacial acetic acid and the solvent removed under reduced pressure. The residue was triturated with water and washed with a mixture of water and acetonitrile (4:1 by volume) to afford title compound as a slightly pink solid, 0.16 g.
¹H NMR (DMSO-d6): δ 2.30 (s, 3H), 3.15 (s, 3H), 3.80 (s, 2H), 6.85 (dd, J = 1.8, 7.4 Hz, 1H), 7.05 (d, J = 8.5 Hz, 1H), 7.75 (d, J = 8.5 Hz, 2H), 8.10 (br s, 1H), 8.30 (d, J = 7.4 Hz, 1H).
MS: ESI (+ve) (Method A): 444 (M+H)⁺, Retention time 11.0 min.
MS: ESI (+ve) (Method B): 444 (M+H)⁺, Retention time 3.6 min.

### Example 13 and 14: [6-cyano-3-(4-methanesulfonylphenylsulfanyl)-2-methylindolizin-1-yl]acetic acid and [6-carbamoyl-3-(4-methanesulfonylphenylsulfanyl)-2-methylindolizin-1-yl]acetic acid

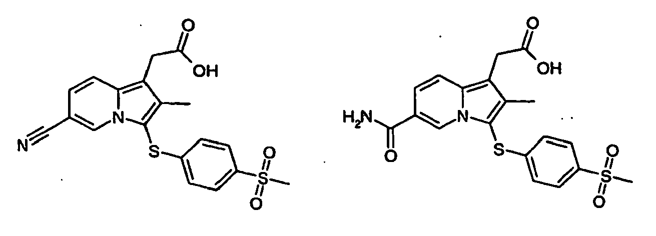

### Preparation 13a and 14a: 3-(5-cyanopyridin-2-yl)propionic acid ethyl ester

A solution of 3-ethoxy-3-oxopropylzinc bromide in tetrahydrofuran (0.5 M, 60 mL) was added dropwise over a period of 45 minutes to a mixture of 6-bromonicotinonitrile (5.0 g), tetrakis(triphenylphosphine)palladium(0), (0.69 g) and tetrahydrofuran (20 mL) at room temperature and the resulting mixture was stirred at room temperature for 6 hours. The mixture was diluted with water (150 mL), basified by the addition of saturated aqueous sodium hydrogen carbonate solution and extracted with methyl *tert*-butyl ether. The combined extracts were dried over magnesium sulfate and the solvent removed under reduced pressure. Purification of the residue by column chromatography on silica gel, eluting with a mixture of pentane, dichloromethane and ethyl acetate (2:1:0, 0:1:0 and 0:5:1 by volume), gave title compound as a pale yellow oil, 4.1 g.
¹H NMR (CDCl₃): δ 1.25 (m, 3H), 2.85 (t, J = 7.2 Hz, 2H), 3.2 (t, J = 7.2 Hz, 2H), 4.15 (m, 2H), 7.35 (d, J = 8.0 Hz, 1H), 7.85 (m, 1H), 8.80 (s, 1H).
MS: ESI (+ve) (Method B): 205 (M+H)⁺, Retention time 2.7 min.

### Preparation 13b and 14b: (6-cyano-2-methylindolizin-1-yl)acetic acid ethyl ester

A mixture of 3-(5-cyanopyridin-2-yl)propionic acid ethyl ester (3.2 g), 1-bromopropan-2-one (5.3 g mL), sodium hydrogen carbonate (3.9 g) and acetonitrile (40 mL) was heated at reflux for 41 hours. The mixture was cooled to room temperature, diluted with water and extracted with ethyl acetate. The combined extracts were dried over magnesium sulfate and the solvent removed under reduced pressure. Purification of the residue by column chromatography on silica gel, eluting with a mixture of dichloromethane and pentane (1:4 to 1:1 by volume), gave title compound as a yellow solid, 0.51 g.
¹H NMR (CDCl₃): δ 1.25 (t, J = 7.1 Hz, 3H), 2.30 (s, 3H), 3.65 (s, 2H), 4.10 (q, J = 7.1 Hz, 2H), 6.65 (dd, J = 1.4, 9.3 Hz, 1H), 7.20 (m, 1H), 7.35 (dd, J = 0.9, 9.3 Hz, 1H), 8.20 (m, 1H).
MS: ESI (+ve) (Method B): 443 (M+H)⁺, Retention time 3.5 min.

### Preparation 13c and 14c: [6-cyano-3-(4-methanesulfonylphenylsulfanyl)-2-methylindolizin-1-yl]acetic acid ethyl ester

Sulfuryl chloride (0.057 mL) was added to a solution of bis(4-methylsulfonylphenyl) disulfide (0.32 g) in dichloromethane (10 mL) at 0 °C and the resulting solution was stirred at room temperature for 1 hour. This solution was added to a solution of (6-cyano-2-methylindolizin-1-yl)acetic acid ethyl ester (0.17 g) in *N,N-*dimethylformamide (1.0 mL) at room temperature and the resulting solution was stirred at room temperature for 15 minutes. The mixture was washed with saturated aqueous sodium hydrogen carbonate solution and saturated aqueous sodium chloride solution, dried over magnesium sulfate and the solvent removed under reduced pressure. Purification of the residue by column chromatography on silica gel, eluting with a mixture of pentane, dichloromethane and ethyl acetate (1:3:0 to 0:1:0 to 0:10:1 by volume), gave title compound as a yellow solid, 0.17 g.
MS: ESI (+ve) (Method B): 429 (M+H)⁺, Retention time 3.8 min.

### Preparation 13d and 14d: [6-cyano-3-(4-methanesulfonylphenylsulfanyl)-2-methylindolizin-1-yl]acetic acid and [6-carbamoyl-3-(4-methanesulfonylphenylsulfanyl)-2-methylindolizin-1-yl]acetic acid

A mixture of [6-cyano-3-(4-methanesulfonylphenylsulfanyl)-2-methylindolizin-1-yl]acetic acid ethyl ester (0.17 g), ethanol (8.0 mL), water (1.0 mL) and 5.0 M aqueous lithium hydroxide solution (0.5 mL) was stirred at 50 °C for 1 hours. The mixture was cooled to room temperature, acidified by the addition of glacial acetic acid and the solvent removed under reduced pressure. The residue was purified by preparative reverse-phase HPLC using a gradient over 30 minutes of acetonitrile in water (30 % to 95 % of organic modifier) to afford [6-cyano-3-(4-methanesulfonylphenylsulfanyl)-2-methylindolizin-1-yl]acetic acid as a pale yellow solid, 0.047 g and [6-carbamoyl-3-(4-methanesulfonylphenylsulfanyl)-2-methylindolizin-1-yl]acetic acid as a pale yellow solid, 0.088 g.

### [6-cyano-3-(4-methanesulfonylphenylsulfanyl)-2-methylindolizin-1-yl]acetic acid

¹H NMR (DMSO-d6): δ 2.30 (s, 3H), 3.15 (s, 3H), 3.80 (s, 2H), 7.05 (d, J = 8.6 Hz, 2H), 7.10 (dd, J = 1.4, 9.2 Hz, 1H), 7.75 (dd, J = 0.7, 9.2 Hz, 1H), 7.80 (d, J = 8.6 Hz, 2H), 8.95 (m, 1H), 12.40 (br s, 1H).
MS: ESI (+ve) (Method A): 401 (M+H)⁺, Retention time 9.0 min.
MS: ESI (+ve) (Method B): 401 (M+H)⁺, Retention time 3.2 min.

### [6-carbamoyl-3-(4-methanesulfonylphenylsulfanyl)-2-methylindolizin-1-yl]acetic acid

¹H NMR (DMSO-d6): δ 2.30 (s, 3H), 3.15 (s, 3H), 3.80 (s, 2H), 7.05 (d, J = 8.7 Hz, 2H), 7.40 (dd, J = 1.4, 9.2 Hz, 1H), 7.65 (d, J = 9.2 Hz, 1H), 7.75 (d, J = 8.7 Hz, 2H), 8.05 (br s, 2H), 8.75 (m, 1H), 12.30 (br s, 1H).
MS: ESI (+ve) (Method A): 419 (M+H)⁺, Retention time 7.2 min.
MS: ESI (+ve) (Method B): 419 (M+H)⁺, Retention time 2.4 min.

### Example 15 and 16: [3-(4-methanesulfonylphenylsulfanyl)-2-methyl-6-trifluoromethylindolizin-1-yl]acetic acid and [7-chloro-3-(4-methanesulfonylphenylsulfanyl)-2-methyl-6-trifluoromethylindolizin-1-yl]acetic acid

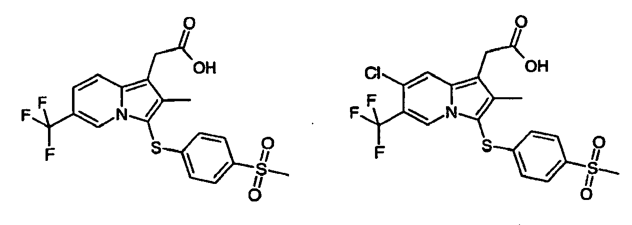

### Preparation 15a and 16a: 3-(5-trifluoromethylpyridin-2-yl)propionic acid ethyl ester

A solution of 3-ethoxy-3-oxopropylzinc bromide in tetrahydrofuran (0.5 M, 63 mL) was added dropwise over a period of 20 minutes to a mixture of 2-bromo-5-trifluoromethylpyridine (4.8 g), tetrakis(triphenylphosphine)palladium(0), (1.0 g) and toluene (40 mL) at room temperature and the resulting mixture was stirred at room temperature for 24 hour. The mixture was filtered through hyflo and the filtrate concentrated under reduced pressure. The residue was diluted with ethyl acetate, washed with water and saturated aqueous sodium chloride solution and then dried over magnesium sulfate. The solvent was removed under reduced pressure and the residue purified by column chromatography on silica gel, eluting with a mixture of pentane and ethyl acetate (19:1 by volume), to afford title compound as a yellow oil, 4.2 g.
¹H NMR (CDCl₃): δ 1.25 (t, J = 7.2 Hz, 3H), 2.85 (t, J = 7.2 Hz, 2H), 3.20 (t, J = 7.2 Hz, 2H), 4.10 (q, J = 7.2 Hz, 2H), 7.35 (d, J = 8.1 Hz, 1H), 7.80 (dd, J = 1.5, 8.1 Hz, 1H), 8.8(brs,1H).
MS: ESI (+ve) (Method B): 248 (M+H)⁺, Retention time 3.4 min.

### Preparation 15b and 16b: (2-methyl-6-trifluoromethylindolizin-1-yl)acetic acid ethyl ester

A mixture of 3-(5-trifluoromethylpyridin-2-yl)propionic acid ethyl ester (1.6 g), 1-bromopropan-2-one (1.8g), sodium hydrogen carbonate (1.7 g) and acetonitrile (17 mL) was heated at reflux for 24 hours. The mixture was cooled to room temperature and concentrated under reduced pressure. Purification of the residue by column chromatography on silica gel, eluting with a mixture of diethyl ether and pentane (1:19 by volume), gave title compound as a yellow oil, 0.66 g.
MS: ESI (+ve) (Method B): 286 (M+H)⁺, Retention time 4.1 min.

### Preparation 15c and 16c: [3-(4-methanesulfonylphenylsulfanyl)-2-methyl-6-trifluoromethylindolizin-1-yl]acetic acid ethyl ester and [7-chloro-3-(4-methanesulfonylphenylsulfanyl)-2-methyl-6-trifluoromethylindolizin-1-yl]acetic acid ethyl ester

Sulfuryl chloride (0.370 mL) was added to a mixture of bis[4-(methylsulfonyl)phenyl]disulfide (1.2 g) and dichloromethane (30 mL) at 0 °C and the resulting mixture was stirred at room temperature for 90 minutes. A solution of (2-methyl-6-trifluoromethylindolizin-1-yl)acetic acid ethyl ester (0.66 g) in dichloromethane (5.0 mL) was added and the resulting mixture was stirred at room temperature for 24 hours. The mixture was diluted with saturated aqueous sodium hydrogen carbonate solution and the organic phase washed with water and saturated aqueous sodium chloride solution and dried over magnesium sulfate. The solvent was removed under reduced pressure and the residue purified by column chromatography on silica gel, eluting with dichloromethane, to afford title compounds as a yellow/brown solid, 0.48 g.
MS: ESI (+ve) (Method B): 472 (M+H)⁺, Retention time 4.2 min.

### Preparation 15d and 16d: [3-(4-methanesulfonylphenylsulfanyl)-2-methyl-6-trifluoromethylindolizin-1-yl]acetic acid and [7-chloro-3-(4-methanesulfonylphenylsulfanyl)-2-methyl-6-trifluoromethylindolizin-1-yl]acetic acid

A mixture of [3-(4-methanesulfonylphenylsulfanyl)-2-methyl-6-trifluoromethylindolizin-1-yl]acetic acid ethyl ester, [7-chloro-3-(4-methanesulfonylphenylsulfanyl)-2-methyl-6-trifluoromethylindolizin-1-yl]acetic acid ethyl ester (0.48 g), methanol (15 mL) and 1.0 M aqueous sodium hydroxide solution (3.0 mL) was stirred at 50°C for 2 hours. The mixture was cooled to room temperature and concentrated under reduced pressure. The residue was acidified by the addition of 1.0 M aqueous hydrochloric acid, extracted with dichloromethane and the combined extracts washed with saturated aqueous sodium chloride solution and then dried over magnesium sulphate. The solvent was removed under reduced pressure and the residue purified by preparative reverse-phase HPLC using a gradient over 30 minutes of acetonitrile in water (30 % to 70 % of organic modifier) to afford [3-(4-methanesulfonylphenylsulfanyl)-2-methyl-6-trifluoromethylindolizin-1-yl]acetic acid as a pale blue solid, 0.22 g and [7-chloro-3-(4-methanesulfonylphenylsulfanyl)-2-methyl-6-trifluoromethylindolizin-1-yl]acetic acid as a pale blue solid, 0.095 g.

### [3-(4-methanesulfonylphenylsulfanyl)-2-methyl-6-trifluoromethylindolizin-1-yl]acetic acid

¹H NMR (CDCl₃): δ 2.35 (s, 3H), 3.00 (s, 3H), 3.80 (s, 2H), 6.95 (d, J = 8.6 Hz, 2H), 7.00 (dd, J = 1.3, 9.3 Hz, 1H), 7.55 (d, J = 9.3 Hz, 1H), 7.75 (d, J = 8.6 Hz, 2H), 8.45 (m, 1H).
MS: ESI (+ve) (Method A): 444 (M+H)⁺, Retention time 11.1 min.
MS: ESI (+ve) (Method B): 444 (M+H)⁺, Retention time 3.6 min.

### [7-chloro-3-(4-methanesulfonylphenylsulfanyl)-2-methyl-6-trifluoromethylindolizin-1-yl]acetic acid

¹H NMR (CDCl₃): δ 2.35 (s, 3H), 3.00 (s, 3H), 3.75 (s, 2H), 6.95 (d, J = 8.5 Hz, 2H), 7.55 (s, 1H), 7.75 (d, J = 8.5 Hz, 2H), 8.50 (s, 1H).
MS: ESI (+ve) (Method A): 478 (M+H)⁺, Retention time 11.6 min.
MS: ESI (+ve) (Method B): 478 (M+H)⁺, Retention time 3.8 min.

### Example 17: [7-cyano-3-(4-methanesulfonylphenylsulfanyl)-2-methylindolizin-1-yl]acetic acid

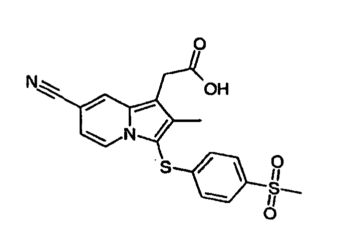

### Preparation 17a: 3-(4-cyanopyridin-2-yl)propionic acid ethyl ester

A solution of 3-ethoxy-3-oxopropylzinc bromide in tetrahydrofuran (0.5 M, 8:0 mL) was added dropwise to a mixture of 2-chloroisonicotinonitrile (0.50 g), tetrakis(triphenylphosphine)palladium(0), (0.21 g) and toluene (8.0 mL) at room temperature and the resulting mixture was stirred at 100°C for 18 hours. The mixture was cooled to room temperature, diluted with saturated aqueous sodium hydrogen carbonate solution and extracted with ethyl acetate. The combined extracts were dried over sodium sulfate and the solvent removed under reduced pressure. Purification of the residue by column chromatography on silica gel, eluting with a mixture of cyclohexane and ethyl acetate (9:1 by volume), gave title compound, 0.44 g.
MS: ESI (+ve) (Method B): 205 (M+H)⁺, Retention time 2.8 min.

### Preparation 17b: (7-cyano-2-methylindolizin-1-yl)acetic acid ethyl ester

A mixture of 3-(4-cyanopyridin-2-yl)propionic acid ethyl ester (0.78 g), 1-bromopropan-2-one (1.3g), sodium hydrogen carbonate (0.96 g) and acetonitrile (6.0 mL) was sealed in a flask and heated at 110 °C for 18 hours. The mixture was cooled to room temperature and the solvent removed under reduced pressure. Purification of the residue by column chromatography on silica gel, eluting with a mixture of cyclohexane and ethyl acetate (19:1 by volume), gave title compound, 0.29 g.
MS: ESI (+ve) (Method B): 243 (M+H)⁺, Retention time 3.6 min.

### Preparation 17c: [7-cyano-3-(4-methanesulfonylphenylsulfanyl)-2-methylindolizin-1-yl]acetic acid ethyl ester

Sulfuryl chloride (0.30 mL) was added to a mixture of bis[4-(methylsulfonyl)phenyl] disulfide (1.6 g) and dichloromethane (30 mL) at 0 °C and the resulting mixture was stirred at room temperature for 90 minutes. A solution of (7-cyano-2-methylindolizin-1-yl)acetic acid ethyl ester (0.29 g) in dichloromethane (1.5 mL) was added and the resulting mixture was stirred at room temperature for 1 hour. The mixture was diluted with saturated aqueous sodium hydrogen carbonate solution and the organic phase dried over magnesium sulfate. The solvent was removed under reduced pressure and the residue purified by column chromatography on silica gel, eluting with a mixture of cyclohexane and ethyl acetate (9:1 by volume), to afford title compound, 0.38 g.
MS: ESI (+ve) (Method B): 428 (M+H)⁺, Retention time 3.9 min.

### Preparation 17d: [7-cyano-3-(4-methanesulfonylphenylsulfanyl)-2-methylindolizin-1-yl]acetic acid

A mixture of [7-cyano-3-(4-methanesulfonylphenylsulfanyl)-2-methylindolizin-1-yl]acetic acid ethyl ester (0.38 g), tetrahydrofuran (30 mL) water (30 mL) and lithium hydroxide (0.12 g) was stirred at room temperature for 2 hours. The mixture was acidified by the addition of 1.0 M aqueous hydrochloric acid and the resulting precipitate collected by filtration and purified by preparative reverse-phase HPLC using a gradient over 30 minutes of acetonitrile in water to afford title compound, 0.080 g
¹H NMR (DMSO-d6): δ 2.30 (s, 3H), 3.15 (s, 3H), 3.85 (s, 2H), 6.90 (dd, J =1.7, 7.3 Hz, 1H), 7.05 (d, J = 8.5 Hz, 2H), 7.80 (d, J = 8.5 Hz, 2H), 8.25 (d, J = 7.3 Hz, 1H), 8.40 (br s, 1H).
MS: ESI (+ve) (Method A): 401 (M+H)⁺, Retention time 9.1 min.

### Example 18 and 19: {6-Fluoro-2-methyl-3-[4-(morpholine-4-sulfonyl)phenylsulfanyl]indolizin-1-yl}acetic acid and {5-chloro-6-fluoro-2-methyl-3-[4-(morpholine-4-sulfonyl)phenylsulfanyl]indolizin-1-yl}acetic acid

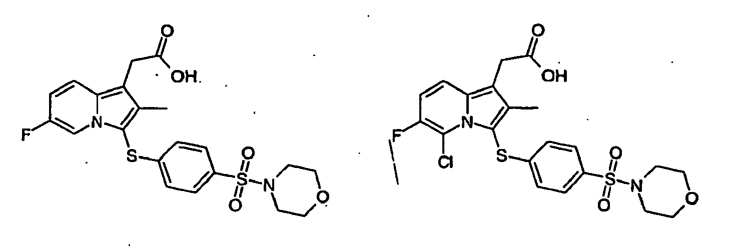

### Preparation 18a and 19a: 4-(4-fluorobenzenesulfonyl)morpholine

A solution of 4-fluorobenzenesulfonyl chloride (6.0 g) in dichloromethane (40 mL) was added dropwise over a period of 10 minutes to a solution of morpholine (8.0 mL) in dichloromethane (40 mL) at 0 °C and the resulting mixture was stirred at 0 °C for 10 minutes and then at room temperature for 10 minutes. The mixture was washed with water, dried over magnesium sulfate and solvent removed under reduced pressure to afford title compound as a white solid, 7.5 g.
¹H NMR (CDCl₃): δ 3.00 (t, J = 4.9 Hz, 4H), 3.75 (t, J = 4.9 Hz, 4H), 7.25 (t, J = 8.4 Hz, 2H), 7.80 (m, 2H).

### Preparation 18b and 19b: bis[4-(morpholine-4-sulfonyl)benzene]disulfide

A mixture of 4-(4-fluorobenzenesulfonyl)morpholine (0.5 g), sodium hydrogensulfide (1.5 g) and 1-methylpyrrolidin-2-one (2.0 mL) was stirred at 80°C for 90 minutes and then at room temperature for 5 hours. The mixture was filtered and the filtrated extracted with ethyl acetate. The aqueous phase was acidified by the addition of concentrated hydrochloric acid, extracted with ethyl acetate and the combined extracts dried over magnesium sulphate. The solvent was removed under reduced pressure and the residue triturated with water to afford title compound as an off-white solid, 0.56 g.
¹H NMR (CDCl₃): δ 3.00 (t, J = 4.7 Hz, 8H), 3.75 (t, J = 4.7 Hz, 8H), 7.65 (d, J = 8.6 Hz, 4H), 7.70 (d, J = 8.6 Hz, 4H).
MS: ESI (+ve) (Method B): 517 (M+H)⁺, Retention time 3.6 min.

### Preparation 18c and 19c: {6-fluoro-2-methyl-3-[4-(morpholine-4-sulfonyl)phenylsulfanyl]indolizin-1-yl}acetic acid ethyl ester and {5-chloro-6-fluoro-2-methyl-3-[4-(morpholine-4-sulfonyl)phenylsulfanyl]indolizin-1-yl}acetic acid ethyl ester

Sulfuryl chloride (0.034 mL) was added to a solution of bis[4-(morpholine-4-sulfonyl)benzene]disulfide (0.26 g) in dichloromethane (5.0 mL) at 0 °C and the resulting solution was stirred at room temperature for 1 hour. A solution of (6-fluoro-2-methylindolizin-1-yl)acetic acid ethyl ester (0.10 g) in dichloromethane (1.0 mL) was added and the resulting solution was stirred at room temperature for 30 minutes. The solution was washed with saturated aqueous sodium hydrogen carbonate solution and saturated aqueous sodium chloride solution, dried over magnesium sulfate and the solvent removed under reduced pressure. Purification of the residue by column chromatography on silica gel, eluting with a mixture of toluene, dichloromethane and ethyl acetate (1:3:0 to 0:1:0 to 0:4:1 by volume), gave title compounds as a brown glass, 0.037 g.
MS: ESI (+ve) (Method B): 493 (M+H)⁺, Retention time 4.2 min.

### Preparation 18d and 19d: {6-fluoro-2-methyl-3-[4-(morpholine-4-sulfonyl)phenylsulfanyl]indolizin-1-yl}acetic acid and {5-chloro-6-fluoro-2-methyl-3-[4-(morpholine-4-sulfonyl)phenylsulfanyl]indolizin-1-yl}acetic acid

A mixture of {6-fluoro-2-methyl-3-[4-(morpholine-4-sulfonyl)phenylsulfanyl]indolizin-1-yl}acetic acid ethyl ester, {5-chloro-6-fluoro-2-methyl-3-[4-(morpholine-4-sulfonyl)phenylsulfanyl]indolizin-1-yl}acetic acid ethyl ester (0.037 g), ethanol (1.0 mL), water (0.1 mL) and 5.0 M aqueous lithium hydroxide solution (0.0455 mL) was stirred at room temperature for 2 hours. The mixture was acidified by the addition of glacial acetic acid and the solvent removed under reduced pressure. Purification of the residue by preparative reverse-phase HPLC using a gradient over 30 minutes of acetonitrile in water (40 % to 95 % of organic modifier) gave {6-fluoro-2-methyl-3-[4-(morpholine-4-sulfonyl)phenylsulfanyl]indolizin-1-yl}acetic acid as a pale green solid, 0.026 g, and {5-chloro-6-fluoro-2-methyl-3-[4-(morpholine-4-sulfonyl)phenylsulfanyl]indolizin-1-yl}acetic acid as a pale yellow solid, 0.0019 g.

### {6-fluoro-2-methyl-3-[4-(morpholine-4-sulfonyl)phenylsulfanyl]indolizin-1-yl}acetic acid

¹H NMR (CD₃OD): δ 2.35 (s, 3H), 2.90 (m, 4H), 3.65 (m, 4H), 3.80 (s, 2H), 6.90 (m, 1H), 7.05 (d, J = 8.5 Hz, 2H), 7.55 (m, 1H), 7.60 (d, J = 8.5 Hz, 2H), 8.15 (dd, J = 1.7, 5.2 Hz, 1H).
MS: ESI (+ve) (Method A): 465 (M+H)⁺, Retention time 10.4 min.
MS: ESI (+ve) (Method B): 465 (M+H)⁺, Retention time 3.5 min.

### (5-chloro-6-fluoro-2-methyl-3-[4-(morpholine-4-sulfonyl)phenylsulfanyl]indolizin-1-yl)-acetic acid

¹H NMR (CD₃OD): δ 2.30 (s, 3H), 2.90 (m, 4H), 3.65 (m, 4H), 3.80 (s, 2H), 6.95 (dd, J = 7.7, 9.6 Hz, 1H), 7.05 (dd, J = 8.6 Hz, 2H), 7.55 (dd, J = 5.3, 9.6 Hz, 1H), 7.60 (d, J = 8.6 Hz, 2H).
MS: ESI (+ve) (Method A): 499 (M+H)⁺, Retention time 10.8 min.
MS: ESI (+ve) (Method B): 499 (M+H)⁺, Retention time 3.6 min.

### Example 20: [6-fluoro-2-methyl-3-(4-methylsulfamoylphenylsulfanyl)indolizin-1-yl]acetic acid

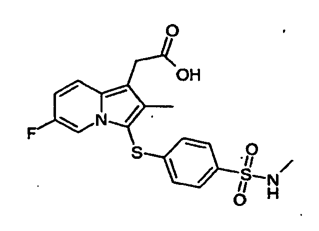

### Preparation 20a: 4-fluoro-N-methylbenzenesulfonamide

A solution of 4-fluorobenzenesulfonyl chloride (9.0 g) in dichloromethane (50 mL) was added dropwise over a period of 10 minutes to a mixture methylamine (10 mL, 40 % wt. in water) in dichloromethane (50 mL) at 0 °C and the resulting mixture was stirred at 0 °C for 10 minutes and then at room temperature for 10 minutes. The mixture was washed with water, dried over magnesium sulfate and solvent removed under reduced pressure to afford title compound as a white solid, 8.2 g.
¹H NMR (CDCl₃): δ 2.65 (d, J = 5.2 Hz, 3H), 4.65 (m, 1H), 7.20 (m, 2H), 7.90 (m, 2H).

### Preparation 20b: 4,4'-dithiobis(N-methylbenzenesulfonamide)

A mixture of 4-fluoro-*N*-methylbenzenesulfonamide (0.5 g), sodium hydrogensulfide (2.0 g) and 1-methylpyrrolidin-2-one (2.5 mL) was stirred at 80 °C for 90 minutes and then at room temperature for 4 hours. The mixture was diluted with water, acidified by the addition of concentrated hydrochloric acid and extracted with dichloromethane. The combined organics were dried over magnesium sulfate and the solvent removed under reduced pressure. The residue was triturated with water to afford title compound as a white solid, 0.20 g.
MS: ESI (-ve) (Method B): 403 (M-H)⁻, Retention time 3.2 min.

### Preparation 20c: [6-fluoro-2-methyl-3-(4-methylsulfamoylphenylsulfanyl)indolizin-1-yl]acetic acid ethyl ester

Sulfuryl, chloride (0.031 mL) was added to a mixture of 4,4'-dithiobis(*N-*methylbenzenesulfonamide) (0.19 g) in dichloromethane (5.0 mL) at 0 °C and the resulting mixture was stirred at room temperature for 1 hour. A solution (6-fluoro-2-methylindolizin-1-yl)acetic acid ethyl ester (0.092 g) in dichloromethane (1.0 mL) was added and the resulting mixture stirred at room temperature for 30 minutes. The mixture was diluted with dichloromethane, washed with saturated aqueous sodium hydrogen carbonate solution and saturated aqueous sodium chloride solution and then dried over magnesium sulfate. The solvent was removed under reduced pressure and the residue purified by column chromatography on silica gel, eluting with a mixture of toluene, dichloromethane and ethyl acetate (1:3:0 to 0:1:0 to 0:4:1 by volume), to afford title compound as a brown glass, 0.022 g.
MS: ESI (+ve) (Method B): 437 (M+H)⁺, Retention time 3.9 min.

### Preparation 20d: [6-fluoro-2-methyl-3-(4-methylsulfamoylphenylsulfanyl)indolizin-1-yl]acetic acid

A mixture of [6-fluoro-2-methyl-3-(4-methylsulfamoylphenylsulfanyl)indolizin-1-yl]acetic acid ethyl ester (0.022 g), ethanol (1:0 mL), water (0.1 mL) and 5.0 M aqueous lithium hydroxide solution (0.03 mL) was stirred at room temperature for 2 hours. The mixture was acidified by the addition of glacial acetic acid and the solvent removed under reduced pressure. Purification of the residue by preparative reverse-phase HPLC using a gradient over 30 minutes of acetonitrile in water (40 % to 95 % of organic modifier) gave title compound as a pale blue/green solid, 0.014g.
¹H NMR (CD₃OD): δ 2.35 (s, 3H), 2.45 (s, 3H), 3.80 (s, 2H), 6.85 (m, 1H), 7.00 (d, J = 8.6 Hz, 2H), 7.55 (dd, J =5.2, 9.7 Hz, 1H), 7.65 (d, J = 8.6 Hz, 2H), 8.10 (dd, J = 2.0, 5.5 Hz, 1H).
MS: ESI (+ve) (Method A): 409 (M+H)⁺, Retention time 9.7 min.
MS: ESI (+ve) (Method B): 409 (M+H)⁺, Retention time 3.3 min.

### Example 21 and 22: [3-(2-chloro-4-methanesulfonylphenylsulfanyl)-6-fluoro-2-methylindolizin-1-yl]acetic acid and [7-chloro-3-(2-chloro-4-methanesulfonylphenylsulfanyl)-6-fluoro-2-methylindolizin-1-yl]acetic acid

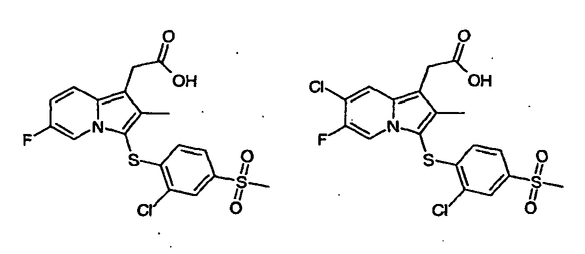

### Preparation 21a and 22a: 2-chloro-1-fluoro-4-methanesulfonylbenzene

A solution of sodium nitrite (5.7 g) in water (15 mL) was added dropwise over a period 15 minutes to a mixture of 2-fluoro-5-methanesulfonylphenylamine (15 g), concentrated hydrochloric acid (30 mL) and water (90 mL) at 0-10 °C. The solution was added to a mixture of copper chloride(I) chloride (11.8 g) and concentrated hydrochloric acid (30 mL) at 0 °C and the resulting mixture heated at 40 °C for 10 minutes. The mixture was cooled to room temperature, filtered and the filtrate extracted with dichloromethane. The combined extracts were dried over magnesium sulfate and the solvent removed under reduced pressure. Purification of the residue by column chromatography on silica gel, eluting with a mixture of toluene, dichloromethane and ethyl acetate (2:1:0 to 0:1:0 to 0:50:1 by volume) gave title compound as a white solid, 3.7 g.
¹H NMR (CDCl₃): δ 3.05 (s, 3H), 7.35 (t, J = 8.4 Hz, 1H), 7.85 (m, 1H), 8.05 (dd, J = 2.2, 6.6 Hz, 1H).

### Preparation 21 b and 22b: bis(2-chloro-4-methanesulfonylbenzene)disulfide

A mixture of 2-chloro-1-fluoro-4-methanesulfonylbenzene (1.0 g), sodium hydrogensulfide (3.6 g) and 1-methylpyrrolidin-2-one (5.0 mL) was stirred at 80 °C for 90 minutes and then at room temperature for 4 hours. The mixture was diluted with water (40 mL) and acidified by the addition of concentrated hydrochloric acid. The resulting precipitate was collected by filtration, washed with water and ethanol and then crystallised from toluene to give title compound as a white solid, 0.61 g.
¹H NMR (DMSO-d6): δ 3.30 (s, 6H), 7.80 (d, J = 8.3 Hz, 2H), 7.90 (dd, J = 1.5, 8.3 Hz, 2H), 8.10 (br s, 2H).
MS: ESI (+ve) (Method B): 443 (M+H)⁺, Retention time 3.8 min.

### Preparation 21c and 22c: [3-(2-chloro-4-methanesulfonylphenylsulfanyl)-6-fluoro-2-methylindolizin-1-yl]acetic acid ethyl ester and [7-chloro-3-(2-chloro-4-methanesulfonylphenylsulfanyl)-6-fluoro-2-methylindolizin-1-yl]acetic acid ethyl ester

Sulfuryl chloride (0.044 mL) was added to a mixture of bis(2-chloro-4-methanesulfonylbenzene)disulfide (0.28 g) in dichloromethane (10 mL) at 0 °C and the resulting mixture was stirred at 50 °C for 2 hour. The mixture was cooled to room temperature and a solution (6-fluoro-2-methylindolizin-1-yl)acetic acid ethyl ester (0.10 g) in dichloromethane (1.0 mL) was added and the resulting mixture was stirred at room temperature for 15 hours. The mixture was diluted with dichloromethane, washed with saturated aqueous sodium hydrogen carbonate solution and dried over magnesium sulfate. The solvent was removed under reduced pressure and the residue purified by column chromatography on silica gel, eluting with a mixture of toluene, dichloromethane and ethyl acetate (1:3:0 to 0:1:0 to 0:8:1 by volume), to afford title compounds as a brown gum, 0.037 g.
MS: ESI (+ve) (Method B): 456 (M+H)⁺, Retention time 4.3 min.

### Preparation 21d and 22d: [3-(2-chloro-4-methanesulfonylphenylsulfanyl)-6-fluoro-2-methylindolizin-1-yl]acetic acid and [7-chloro-3-(2-chloro-4-methanesulfonylphenylsulfanyl)-6-fluoro-2-methylindolizin-1-yl]acetic acid

A mixture of [3-(2-chloro-4-methanesulfonylphenylsulfanyl)-6-fluoro-2-methylindolizin-1-yl]acetic acid ethyl ester, [7-chloro-3-(2-chloro-4-methanesulfonylphenylsulfanyl)-6-fluoro-2-methylindolizin-1-yl]acetic acid ethyl ester (0.037 g), ethanol (3.0 mL), water (0.2 mL) and 5.0 M aqueous lithium hydroxide solution (0.12 mL) was stirred at room temperature for 4 hours. The mixture was acidified by the addition of glacial acetic acid and the solvent removed under reduced pressure. Purification of the residue by preparative reverse-phase HPLC using a gradient over 30 minutes of acetonitrile in water (40 % to 95 % of organic modifier) gave [3-(2-chloro-4-methanesulfonylphenylsulfanyl)-6-fluoro-2-methylindolizin-1-yl]acetic acid as a pale green solid, 0.027 g and [7-chloro-3-(2-chloro-4-methanesulfonylphenylsulfanyl)-6-fluoro-2-methylindolizin-1-yl]acetic acid as a yellow solid, 0.0019 g.

### [3-(2-chloro-4-methanesulfonylphenylsulfanyl)-6-fluoro-2-methylindolizin-1-yl]acetic acid

¹H NMR (CD₃OD): δ 2.30 (s, 3H), 3.10 (s, 3H), 3.80 (s, 2H), 6.30 (d, J = 8.5 Hz, 1H), 6.90 (m, 1H), 7.55-7.60 (m, 2H), 8.00 (d, J = 1.9 Hz, 1H), 8.05 (dd, J = 1.9, 5.2 Hz, 1H).
MS: ESI (+ve) (Method A): 428 (M+H)⁺, Retention time 10.6 min.
MS: ESI (+ve) (Method B): 428 (M+H)⁺, Retention time 3.6 min.

### [7-chloro-3-(2-chloro-4-methanesulfonylphenylsulfanyl)-6-fluoro-2-methylindolizin-1-yl]acetic acid

¹H NMR (CD₃OD): δ 2.30 (s, 3H), 3.10 (s, 3H), 3.80 (s, 2H), 6.35 (d, J = 8.4 Hz, 1H), 7.55 (dd, J = 1.9, 8.4 Hz, 1H), 7.75 (d, J = 7.1 Hz, 1H), 8.00 (d, J = 1.9 Hz, 1H), 8.20 (d, J = 5.2Hz, 1H).
MS: ESI (+ve) (Method A): 462 (M+H)⁺, Retention time 11.3 min.
MS: ESI (+ve) (Method B): 462 (M+H)⁺, Retention time 3.8 min.

### Examples 23: [3-(3-chloro-4-methanesulfonyl-phenylsulfanyl)-6-fluoro-2-methylindolizin-1-yl]acetic acid

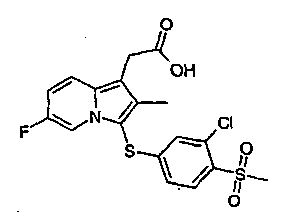

### Preparation 23a: 2-chloro-4-fluoro-1-methanesulfonylbenzene

3-Chloroperoxybenzoic acid (32 g) was added portionwise to a solution of 2-chloro-4-fluoro-1-methylsulfanylbenzene (11.5 g) in dichloromethane (300 mL) at room temperature and the resulting mixture was stirred at room temperature for 2 hours. The mixture was filtered and the filtrated washed with saturated aqueous sodium thiosulfate solution and saturated aqueous sodium hydrogen carbonate solution and then dried over magnesium sulfate. The solvent was removed under reduced pressure and the residue triturated with diethyl ether to afford title compound as a sandy solid, 8.4 g.
¹H NMR (CDCl₃): δ 3.25 (s, 3H), 7.20 (m, 1H), 7.30 (dd, J = 2.4, 8.2, 1H), 8.20 (dd, J = 5.9, 8.9 Hz, 1H).

### Preparation 23b: bis(3-chloro-4-methanesulfonylbenzene)disulfide

A mixture of 2-chloro-4-fluoro-1-methanesulfonylbenzene (1.0 g), sodium hydrogensulfide (3.6 g) and 1-methylpyrrolidin-2-one (5.0 mL) was stirred at 80 °C for 90 minutes and then at room temperature for 4 hours. The mixture was diluted with water (40 mL), acidified by the addition of concentrated hydrochloric acid and extracted with dichloromethane. The combined extracts were dried over magnesium sulfate and the solvent removed under reduced pressure. The residue was triturated with water and the resulting solid crystallised from toluene to afford title compound as a white crystalline solid, 0.69 g.
¹H NMR (DMSO-d6): δ 3.35 (s, 6H), 7.80 (dd, J = 2.0, 8.4 Hz, 2H), 7.95 (d, J = 2.0 Hz, 2H), 8.05 (d, J = 8.4 Hz, 2H).
MS: ESI (+ve) (Method B): 443 (M+H)⁺, Retention time 3.6 min.

### Preparation 23c: [3-(3-chloro-4-methanesulfonylphenylsulfanyl)-6-fluoro-2-methylindolizin-1-yl]acetic acid ethyl ester

Sulfuryl chloride (0.044 mL) was added to a solution of bis(3-chloro-4-methanesulfonylbenzene)disulfide (0.28 g) in dichloromethane (10 mL) at 0 °C and the resulting solution was stirred at room temperature for 30 minutes and then at 40 °C for 2 hours. The solution was cooled to room temperature and a solution of (6-fluoro-2-methylindolizin-1-yl)acetic acid ethyl ester (0.10 g) in dichloromethane (1.0 mL) was added and the resulting mixture was stirred at room temperature for 17 hours. The mixture was diluted with dichloromethane, washed with saturated aqueous sodium hydrogen carbonate solution and dried over magnesium sulfate. The solvent was removed under reduced pressure and the residue purified by column chromatography on silica gel, eluting with a mixture of toluene, dichloromethane and ethyl acetate (1:3:0 to 0:1:0 to 0:5:1 by volume), to afford title compound as a brown gum, 0.057 g.
MS: ESI (+ve) (Method B): 456 (M+H)⁺, Retention time 4.2 min.

### Preparation 23d: [3-(3-chloro-4-methanesulfonyl-phenylsulfanyl)-6-fluoro-2-methylindolizin-1-yl]acetic acid

A mixture of [3-(3-chloro-4-methanesulfonylphenylsulfanyl)-6-fluoro-2-methylindolizin-1-yl]acetic acid ethyl ester (0.057 g), ethanol (1.0 mL), water (0.1 mL) and 5.0 M aqueous lithium hydroxide solution (0.072 mL) was stirred at room temperature for 2 hours. The mixture was acidified by the addition of glacial acetic acid and the solvent removed under reduced pressure. Purification of the residue by preparative reverse-phase HPLC using a gradient over 30 minutes of acetonitrile in water (40 % to 95 % of organic modifier) gave title compound as a pale green solid, 0.016 g.
¹H NMR (CD₃OD): δ 2.35 (s, 3H), 3.20 (s, 3H), 3.80 (s, 2H), 6.90 (m, 2H), 7.05 (d, J = 1.9 Hz, 1H), 7.55 (dd, J = 5.4, 9.6 Hz, 1H), 7.90 (d, J = 8.4 Hz, 1H), 8.15 (dd, J = 1.9, 5.2 Hz, 1H).
MS: ESI (+ve) (Method A): 428 (M+H)⁺, Retention time 10.2 min.
MS: ESI (+ve) (Method B): 428 (M+H)⁺, Retention time 3.5 min.

### Example 24: [3-(4-ethanesulfonylaminophenylsulfanyl)-6-fluoro-2-methylindolizin-1-yl]acetic acid

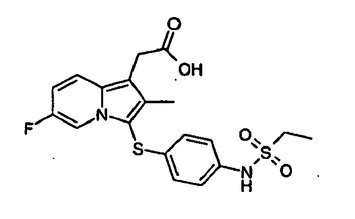

### Preparation 24a: ethanesulfonic acid [4-(4-ethanesulfonylaminophenyldisulfanyl)phenyl]amide

A solution of ethanesulfonyl chloride (1.9 mL) in dichloromethane (5.0 mL) was added dropwise to a solution of 4-aminophenyl disulfide (2.0 g) and triethylamine (4.5 mL) in dichloromethane (20 mL) at -40 °C and the resulting mixture was warmed to room temperature over a period of 4 hours. The mixture was washed with 1.0 M aqueous sodium hydroxide solution and the aqueous phase acidified by the addition of 1.0 M aqueous hydrochloric acid and then extracted with ethyl acetate. The combined extracts were dried over magnesium sulfate and solvent removed under reduced pressure to afford title compound as a whte solid, 2.0 g.
¹H NMR (DMSO-d6): δ 1.15 (t, J = 7.4 Hz, 6H), 3.10 (q, J = 7.4 Hz, 4H), 7.20 (m, 4H), 7.45 (m, 4H), 9.85 (br s, 2H).

### Preparation 24b: [3-(4-ethanesulfonylaminophenylsulfanyl)-6-fluoro-2-methylindolizin-1-yl]acetic acid ethyl ester

Sulfuryl chloride (0.040 mL) was added to a mixture of ethanesulfonic acid [4-(4-ethanesulfonylaminophenyldisulfanyl)phenyl]amide (0.27 g) and dichloromethane (3.0 mL) at room temperature and the resulting mixture was stirred at room temperature for 90 minutes. A solution of (6-fluoro-2-methylindolizin-1-yl)acetic acid ethyl ester (0.10 g) in dichloromethane (1.0 mL) was added and the resulting mixture was stirred at room temperature for 18 hours. The mixture was diluted with saturated aqueous sodium hydrogen carbonate solution and the organic phase washed with water and saturated aqueous sodium chloride solution and then dried over magnesium sulfate. The solvent was removed under reduced pressure and the residue purified by column chromatography on silica gel, eluting with a mixture of ethyl acetate and pentane (1:3 by volume), to afford title compound as a light brown oil, 0.11 g.
MS: ESI (+ve) (Method B): 451 (M+H)⁺, Retention time 4.1 min.

### Preparation 24c: [3-(4-ethanesulfonylaminophenylsulfanyl)-6-fluoro-2-methylindolizin-1-yl]acetic acid

A mixture of [3-(4-ethanesulfonylaminophenylsulfanyl)-6-fluoro-2-methylindolizin-1-yl]acetic acid ethyl ester (0.10 g), methanol (2.0 mL) and 1.0 M aqueous sodium hydroxide solution (0.5 mL) was stirred at room temperature for 2 hours. The mixture was extracted with dichloromethane and the aqueous phase acidified by the addition of 1.0 M aqueous hydrochloric acid and then extracts with dichloromethane. The combined extracts were dried over magnesium sulfate and the solvent removed under reduced pressure. Purification of the residue by preparative reverse-phase HPLC using a gradient over 30 minutes of acetonitrile in water (50 % to 75 % of organic modifier) gave title compound as a pale blue solid, 0.053 g.
¹H NMR (CDCl₃): δ 1.35 (t, J = 7.4 Hz, 3H), 2.35 (s, 3H), 3.05 (q, J = 7.4 Hz, 2H), 3.80 (s, 2H), 6.40 (br s, 1H), 6.75 (m, 1H), 6.85 (d, J = 8.6 Hz, 2H), 7.05 (d, J = 8.6 Hz, 2H), 7.35 (dd, J = 5.2, 9.7 Hz, 1H), 8.10 (dd, J = 2.0, 5.2 Hz, 1H).
MS: ESI (+ve) (Method A): 423 (M+H)⁺, Retention time 10.0 min.

### Biological Results:

The compounds of the Examples above were tested in the CRTH2 Radioligand Binding assay described above; the compounds had IC₅₀s of less than 300nM in the binding assay. Examples 1-9 were tested in the GTPγS functional assay, and showed IC₅₀ values of <1µM.. Examples 9, 10, 12-16, were tested in the calcium flux functional assay and had IC₅₀ values <3µM

## Claims

1. A compound of formula (I) 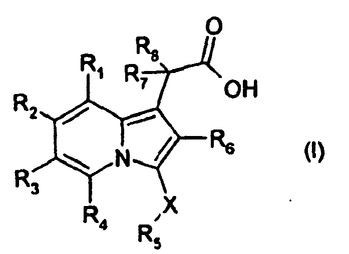 or a salt, N-oxide, hydrate or solvate thereof, for use for the treatment of asthma, chronic obstructive pulmonary disease, rhinitis, allergic airway syndrome or allergic rhinobronchitis.
wherein
R_{1,} R₂, R₃ and R₄ each independently are hydrogen, C₁-C₆alkyl, fully or partially fluorinated C₁-C₆alkyl, halo, -S(O)ₙR₁₀, -SO₂N(R₁₀)₂, -N(R₁₀)₂, -C(O)N(R₁₀)₂, -NR₁₀C(O)R₉, -CO₂R₁₀, -C(O)R₉, -NO₂, -CN or -OR₁₁;
wherein each R₉ is independently methyl, ethyl or phenyl;
R₁₀ is independently hydrogen, methyl, ethyl or phenyl;
R₁₁ is hydrogen. C₁-C₆alkyl, fully or partially fluorinated C₁-C₆alkyl, or -SO₂R₉; and
n is 0, 1 or 2;
R₅ is C₁-C₆alkyl, fully or partially fluorinated C₁-C₆alkyl, C₁-C₆alkenyl, C₁-C₆alkynyl, optionally substituted phenyl, or optionally substituted monocyclic heteroaryl having 5 or 6 ring atoms;
wherein there are up to four optional substituents independently selected from C₁-C₆alkyl, monocyclic saturated C₃-C₈cycloalkyl, C₁-C₆alkoxy, hydroxy, hydroxy(C₁-C₆)alkyl, mercapto, mercapto(C₁-C₆)alkyl, C₁-C₆alkylthio, phenyl, monocyclic heteroaryl having 5 or 6 ring atoms, halo, trifluoromethyl, trifluoromethoxy, nitro, -CN, oxo, -COOH, -COOR^{A}, -COR^{A}, -SO₂R^{A}, -CONH_{2,} -SO₂NH₂, -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂OR^{A}, -NR^{B}SO₂OH, -NR^{B}SO₂OR^{A}, -NHCONH₂, -NR^{A}CONH₂, -NHCONHR^{B}. -NR^{A}CONHR^{B}, -NHCONR^{A}R^{B} or -NR^{A}CONR^{A}R^{B} wherein R^{A} and R^{B} are independently a C₁-C₆alkyl, C₃-C₆ cycloalkyl, phenyl or monocyclic heteroaryl having 5 or 6 ring atoms;
R₆ is hydrogen, C₁-C₈alkyl or fully or partially fluorinated C₁-C₆alkyl;
either R⁷ and R⁸ are independently hydrogen or C₁-C₆alkyl, or R₇ and R₈ together with the atom to which they are attached form a cycloalkyl group; and
X is -CHR₆-, -S(O)ₙ-, -NR₆SO₂- or -SO₂NR₆-, wherein n is 0, 1 or 2.

2. A compound as claimed in claim 1, independent of use, with the proviso that, when X is -CH₂-, R₆ is methyl and R₅ is 4-chlorophenyl, then R₁, R₂, R₃, R₄, R₇ and R₈ are not all hydrogen.

3. A compound as claimed in claim 1 or claim 2, wherein R₁, R₂, R₃ and R₄ are independently selected from hydrogen, methyl, ethyl, trifluoromethyl, fluoro, chloro, bromo, -NO₂, -CN, -SO₂R₉, -SO₂N(R₁₀)₂, -C(O)N(R₁₀)₂, -NR₁₀C(O)R₉, -CO₂R₁₀ and -C(O)R₉, wherein R₉ and R₁₀ are as defined in claim 1.

4. A compound as claimed in any preceding claim, wherein any R₁₁ is selected from methyl, trifluoromethyl, ethyl, phenyl and -SO₂CH₃.

5. A compound as claimed in claim 1 or claim 2, wherein R₁, R₂, R₃ and R₄ are independently selected from hydrogen, chloro, fluoro, cyano, methyl and trifluoromethyl.

6. A compound as claimed in claim 5, wherein at least two of R₁, R₂, R₃ and R₄ are hydrogen.

7. A compound as claimed in any preceding claim, wherein R₅ is methyl, ethyl, n- or iso-propyl, trifluoromethyl, allyl, optionally substituted phenyl, or optionally substituted monocyclic heteroaryl having 5 or 6 ring atoms.

8. A compound as claimed in claim 7, wherein R₅ is optionally substituted pyridyl, pyrimidinyl, furyl, thienyl, imidazolyl, oxazolyl, isoxazolyl, or pyrrolyl.

9. A compound as claimed in claim 7, wherein R₅ is optionally substituted phenyl.

10. A compound as claimed in any of claims 7 to 9, wherein the optional substituents are selected from chloro, fluoro, methylsulfonyl, ethylsulfonyl, carbamate, methylcarbamate, methylaminosulfonyl, ethylaminosulfonyl, methylsulfonylamino, ethylsulfonylamino, morpholin-1-ylsulfonyl, piperidin-1-ylsulfonyl, piperazin-1-ylsulfonyl, 4-methylpiperazin-1-ylsulfonyl and tetrahydropyrrol-1-ylsulfonyl.

11. A compound as claimed in any preceding claim, wherein X is -CH₂-, -S(O)-, -NHSO₂- or -SO₂NH-.

12. A compound as claimed in any of claims 1 to 10, wherein X is -S- or -SO₂-.

13. A compound as claimed in any preceding claim, wherein R₆ is hydrogen, ethyl or trifluoromethyl.

14. A compound as claimed in any of claims 1 to 12, wherein R₆ is methyl.

15. A compound as claimed in any preceding claim, wherein R₇ and R₈ are each hydrogen.

16. A compound as claimed in any of claims 1 to 14, wherein one of R₇ and R₈ is methyl and the other is hydrogen.

17. A compound as claimed in any of claims 1 to 14, wherein R₇ and R₈ taken together with the carbon atom to which they are attached form a cyclopropyl, cyclopentyl or cyclohexyl ring.

18. A compound as claimed in claim 1 or in claim 2, wherein R₁, R₂, R₃ and R₄ are independently selected from hydrogen, chloro, fluoro, cyano, methyl trifluoromethyl, methoxy, and trifluoromethoxy; X is -S- or -SO₂-; R₅ is optionally substituted phenyl, R₆ is methyl, and R₇ and R₈ are hydrogen.

19. A compound as claimed in claim 18, wherein R₅ is phenyl or phenyl substituted by one or two substituents selected from chloro, fluoro, trifluoromethyl, methylsulfonyl, ethylsulfonyl, carbamate, methylcarbamate, methylaminosulfonyl, ethylaminosulfonyl, methylsulfonylamino, ethylsulfonylamino, morpholin-1-ylsulfonyl, piperidin-1-ylsulfonyl, piperizin-1-ylsulfonyl, 4-methylpiperizin-1-ylsulfonyl, and tetrahydropyrrol-1ylsulfonyl.

20. A pharmaceutical composition comprising a compound as claimed in any of claims 1 to 19, and a pharmaceutically acceptable carrier.

21. Use of a compound as claimed in any of claims 1 to 19, for the manufacture of a composition for the treatment of asthma, chronic obstructive pulmonary disease, rhinitis, allergic airway syndrome or allergic rhinobronchitis.

## Patentansprüche

1. Verbindung der Formel (I) 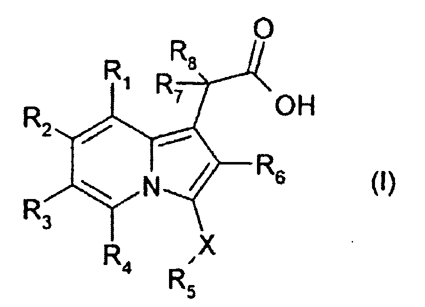 oder Salz, N-Oxid, Hydrat oder Solvat derselben zur Verwendung für die Behandlung von Asthma, chronisch obstruktiver Lungenentzündung, Rhinitis, allergischem Luftwegsyndrom oder allergischer Rhinobronchitis,
wobei
R₁, R₂, R₃ und R₄ jeweils unabhängig Wasserstoff, C₁-C₆-Alkyl, vollständig oder teilweise fluoriertes C₁-C₆-Alkyl, Halo, -S(O)ₙ(R₁₀), -SO₂N(R₁₀)₂, -N(R₁₀)₂, -C(O)N(R₁₀)₂, -NR₁₀C(O)R₉, -CO₂R₁₀, -C(O)R₉, -NO₂, -CN- oder -OR₁₁ sind;
wobei jedes R₉ unabhängig Methyl, Ethyl oder Phenyl ist;
R₁₀ unabhängig Wasserstoff, Methyl, Ethyl oder Phenyl ist;
R₁₁ Wasserstoff, C₁-C₆-Alkyl, vollständig oder teilweise fluoriertes C₁-C₆-Alkyl oder -SO₂R₉ ist; und
n 0, 1 oder 2 beträgt;
R₅ C₁-C₆-Alkyl, vollständig oder teilweise fluoriertes C₁-C₆-Alkyl, C₁-C₆-Alkenyl, C₁-C₆-Alkynyl, wahlweise substituiertes Phenyl oder wahlweise substituiertes monocyclisches Heteroaryl ist, das 5 oder 6 Ringatome aufweist;
wobei bis zu vier wahlweise Substituenten vorliegen, die unabhängig ausgewählt sind unter C₁-C₆-Alkyl, monocyclischem gesättigtem C₃-C₈-Cycloalkyl, C₁-C₆-Alkoxy, Hydroxy, Hydroxy(C₁-C₆)-Alkyl, Mercapto, Mercapto(C₁-C₆)-Alkyl, C₁-C₆-Alkylthio, Phenyl, monocyclischem Heteroaryl, das 5 oder 6 Ringatome aufweist, Halo, Trifluormethyl, Trifluormethoxy, Nitro, -CN, oxo, -COOH, -COOR^{A}, -COR^{A}, -SO₂R^{A}, -CONH₂, -SO₂NH₂, ₋CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂OR^{A}, -NR^{B}SO₂OH, -NR^{B}SO₂OR^{A}, -NHCONH₂, -NR^{A}CONH₂, -NHCONHR^{B}, -NR^{A}CONHR^{B}, -NHCONR^{A}R^{B} oder NR^{A}CONR^{A}R^{B}, wobei R^{A} und R^{B} unabhängig ein C₁-C₆-Alkyl, C₃-C₆-Cycloalkyl, Phenyl oder monocyclisches Heteroaryl sind, das 5 oder 6 Ringatome aufweist;
R₆ Wasserstoff, C₁-C₆-Alkyl oder vollständig oder teilweise fluoriertes C₁-C₆-Alkyl ist;
entweder R₇ oder R₈ unabhängig Wasserstoff oder C₁-C₆-Alkyl sind, oder R₇ und R₈ zusammen mit dem Atom, an das sie angeknüpft sind, eine Cycloalkylgruppe bilden; und
X -CHR₆-, -S(O)ₙ-, -NR₆SO₂- oder -SO₂NR₆- ist, wobei n 0, 1 oder 2 beträgt.

2. Verbindung nach Anspruch 1, unabhängig von der Verwendung, mit der Maßgabe, dass, wenn X -CH₂- ist, R₆ Methyl ist und R₅ 4-Chlorphenyl ist, R₁, R₂, R₃, R₄, R₇, und R₆ dann nicht alle Wasserstoff sind.

3. Verbindung nach Anspruch 1 oder Anspruch 2, wobei R₁, R₂, R₃ unabhängig unter Wasserstoff, Methyl, Ethyl, Trifluormethyl, Fluor, Chlor, Brom, -NO₂, -CN, -SO₂R₉, -SO₂N(R₁₀)₂, C(O)N(R₁₀)₂, -NR₁₀C(O)R₉, -CO₂R₁₀, und -C(O)R₉ aus gewählt werden, wobei R₉ und R₁₀ die in Anspruch 1 aufgeführte Definition aufweisen.

4. Verbindung nach einem der vorhergehenden Ansprüche, wobei irgendein R₁₁ unter Methyl, Trifluormethyl, Ethyl, Phenyl und -SO₂CH₃ ausgewählt wird.

5. Verbindung nach Anspruch 1 oder Anspruch 2, wobei R₁, R₂, R₃ und R₄, unabhängig unter Wasserstoff, Chlor, Fluor, Cyano, Methyl und Trifluormethyl ausgewählt werden.

6. Verbindung nach Anspruch 5, wobei mindestens zwei von R₁, R₂, R₃ und R₄ Wasserstoff sind.

7. Verbindung nach einem der vorhergehenden Ansprüche, wobei R₅ Methyl, Ethyl, n- oder Isopropyl, Trifluormethyl, Allyl, wahlweise substituiertes Phenyl oder wahlweise substituiertes monocyclisches Heteroaryl ist, das 5 oder 6 Ringatome aufweist.

8. Verbindung nach Anspruch 7, wobei R₅ wahlweise substituiertes Pyridyl, Pyrimidinyl, Furyl, Thienyl, Imidazolyl, Oxazolyl, Isoxazolyl oder Pyrrolyl ist.

9. Verbindung nach Anspruch 7, wobei R₅ wahlweise substituiertes Phenyl ist.

10. Verbindung nach einem der Ansprüche 7 bis 9, wobei die wahlweisen Substituenten unter Chlor, Fluor, Methylsulfonyl, Ethylsulfonyl, Carbamat, Methylcarbamat, Methylaminosulfonyl, Ethylaminosulfonyl, Methylsulfonylamino, Ethylsulfonylamino, Morpholin-1-ylsulfonyl, Piperidin-1-ylsulfonyl, Piperazin-1-ylsulfonyl, 4-Methylpiperazin-1-ylsulfonyl und Tetrahydropyrrol-1-ylsulfonyl ausgewählt werden.

11. Verbindung nach einem der vorhergehenden Ansprüche, wobei X -CH₂⁻, -S(O)-, -NHSO₂- oder SO₂NH- ist.

12. Verbindung nach einem der Ansprüche 1 bis 10, wobei X -S- oder -SO₂- ist.

13. Verbindung nach einem der vorhergehenden Ansprüche, wobei R₆ Wasserstoff, Ethyl oder Trifluormethyl ist.

14. Verbindung nach einem der Ansprüche 1 bis 12, wobei R₆ Methyl ist.

15. Verbindung nach einem der vorhergehenden Ansprüche, wobei R₇ und R₈ jeweils Wasserstoff sind.

16. Verbindung nach einem der Ansprüche 1 bis 14, wobei eines von R₇ und R₈ Methyl und das andere Wasserstoff ist.

17. Verbindung nach einem der Ansprüche 1 bis 14, wobei R₇ und R₈ zusammen mit dem Kohlenstoffatom, an das sie angeknüpft sind, einen Cyclopropyl-, Cyclopentyl- oder Cyclohexylring bilden.

18. Verbindung nach Anspruch 1 oder Anspruch 2, wobei R₁, R₂, R₃ und R₄ unabhängig unter Wasserstoff, Chlor, Fluor, Cyano, Methyltrifluormethyl, Methoxy und Trifluormethoxy ausgewählt werden; und X -S- oder -SO₂- ist; R₅ wahlweise substituiertes Phenyl ist, R₆ Methyl ist und R₇ und R₈ Wasserstoff sind.

19. Verbindung nach Anspruch 18, wobei R₅ Phenyl oder Phenyl ist, das durch einen oder zwei Substituenten substituiert ist ausgewählt unter Chlor, Fluor, Trifluormethyl, Methylsulfonyl, Ethylsulfonyl, Carbamat, Methylcarbamat, Methylaminosulfonyl, Ethylaminosulfonyl, Methylsulfonylamino, Ethylsulfonylamino, Morpholin-1-ylsulfonyl, Piperidin-1-ylsulfonyl, Piperazin-1-ylsulfonyl, 4-Methylpiperazin-1-ylsulfonyl und Tetrahydropyrrol-1-ylsulfonyl.

20. Pharmazeutische Zusammensetzung umfassend eine Verbindung nach einem der Ansprüche 1 bis 19 und einen pharmazeutisch akzeptablen Träger.

21. Verwendung einer Verbindung nach einem der Ansprüche 1 bis 19 für die Herstellung einer Zusammensetzung für die Behandlung von Asthma, chronisch obstruktiver Lungenentzündung, Rhinitis, allergischem Luftwegsyndrom oder allergischer Rhinobronchitis.

## Revendications

1. Composé de la formule (I) 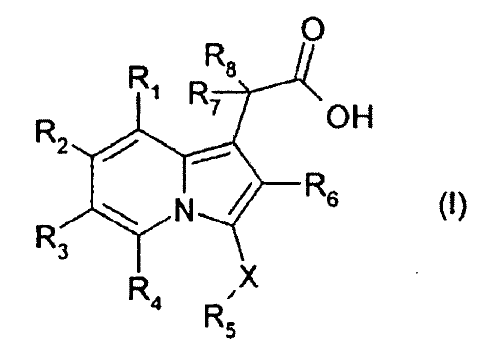 ou son sel, N-oxyde, hydrate ou solvate, destiné à l'utilisation pour le traitement de : asthme, maladie pulmonaire obstructive chronique, rhinite, syndrome allergique des voies respiratoires ou rhino-bronchite allergique,
dans lequel
R₁, R₂, R₃ et R₄ sont chacun indépendamment hydrogène, C₁-C₆alkyle, C₁-C₆alkyle totalement ou partiellement fluoré, halo, -S(O)ₙR₁₀, -SO₂N(R₁₀)₂, -N(R₁₀)₂, -C(O)N(R₁₀)₂, -NR₁₀C(O)R₉, -CO₂R₁₀, -C(O)R₉, -NO₂, -CN ou -OR₁₁ ;
dans lequel chaque R₉ est indépendamment méthyle, éthyle ou phényle ;
R₁₀ est indépendamment hydrogène, méthyle, éthyle ou phényle ;
R₁₁ est hydrogène, C₁-C₆alkyle, C₁-C₆alkyle totalement ou partiellement fluoré, ou - SO₂R₉ ; et
n est 0, 1 ou 2 ;
R₅ est C₁-C₆alkyle, C₁-C₆alkyle totalement ou partiellement fluoré, C₁-C₆alkényle, C₁-C₆alkynyle, phényle optionnellement substitué ou hétéroaryle monocyclique optionnellement substitué ayant 5 ou 6 atomes de noyau ;
dans lequel il y a jusqu'à quatre substituants optionnels sélectionnés indépendamment parmi: C₁-C₆alkyle, C₃-C₈cycloalkyle monocyclique saturé, C₁-C₆alkoxy, hydroxy, hydroxy(C₁-C₆)alkyle, mercapto, mercapto(C₁-C₆)alkyle, C₁-C₆alkylthio, phényle hétéroaryle monocyclique ayant 5 ou 6 atomes de noyau, halo, trifluorométhyle, trifluorométhoxy, nitro, -CN, oxo, -COOH, -COOR^{A}, -COR^{A}, -SO₂R^{A}, -CONH₂, -SO₂NH₂ -CONHR^{A}, -SO₂NHR^{A}, -CONR^{A}R^{B}, -SO₂NR^{A}R^{B}, -NH₂, -NHR^{A}, -NR^{A}R^{B}, -OCONH₂, -OCONHR^{A}, -OCONR^{A}R^{B}, -NHCOR^{A}, -NHCOOR^{A}, -NR^{B}COOR^{A}, -NHSO₂₀R^{A}, -NR⁸SO₂OH, -NR^{B}SO₂OR^{A}, -NHCONH₂, -NR^{A}CONH₂, -NHCONHR^{B}, -NR^{A}CONHR^{B}, -NHCONR^{A}R^{B} ou - NR^{A}CONR ^{A}R dans lequel R^{A} et R^{B} sont indépendamment un C₁-C₆alkyl, C₃-C₆ cycloalkyle, phényle ou hétéroaryle monocyclique ayant 5 ou 6 atomes de noyau ;
R₆ est hydrogène, C₁-C₆alkyle, ou C₁-C₆alkyle totalement ou partiellement fluoré ;
R₇ et R₈ soit sont indépendamment hydrogène ou C₁-C₆alkyle soit forment un groupe cycloalkyle avec l'atome auquel ils sont attachés ; et
X est -CHR₆-, -S(0)ₙ-, -NR₆SO₂- or -SO₂NR₆-, dans lequel n est 0, 1 ou 2.

2. Composé selon la revendication 1, indépendamment de l'utilisation, à condition que, quand X est -CH₂-, R₆ est méthyle et R₅ est 4-chlorophényle, R₁, R₂, R₃, R₄, R₇ et R₈ ne sont alors pas tous hydrogène.

3. Composé selon la revendication 1 ou 2, dans lequel R₁, R₂, R₃ et R₄ sont indépendamment sélectionnés parmi hydrogène, méthyle, éthyle, trifluorométhyle, fluoro, chloro, bromo, -NO₂, -CN, -SO₂R₉, -SO₂N(R₁₀)₂, -C(O)ₙ(R₁₀)₂, -NR₁₀C(O)R₉, -CO₂R₁₀, et -C(O)R₉, dans lequel R₉ et R₁₀ sont selon la revendication 1.

4. Composé selon l'une quelconque des revendications précédentes, dans lequel tout R₁₁ est sélectionné parmi méthyle, trifluorométhyle, éthyle, phényle et -SO₂CH₃.

5. Composé selon la revendication 1 ou 2, dans lequel R₁, R₂, R₃ et R₄ sont indépendamment sélectionnés parmi hydrogène, chloro, fluoro, cyano, méthyle et trifluorométhyle.

6. Composé selon la revendication 5, dans lequel au moins deux parmi R₁, R₂, R₃ et R₄ sont hydrogène.

7. Composé selon l'une quelconque des revendications précédentes, dans lequel R₅ est méthyle, éthyle, n- ou iso-propyle, trifluorométhyle, allyle, phényle optionnellement substitué, ou hétéroaryle monocyclique optionnellement substitué ayant 5 ou 6 atomes de noyau.

8. Composé selon la revendication 7, dans lequel R₅ est pyridyle, pyrimidinyle, furyle, thiényle, imidazolyle, oxazolyle, isoxazolyle, ou pyrrolyle optionnellement substitués.

9. Composé selon la revendication 7, dans lequel R₅ est phényle optionnellement substitué.

10. Composé selon l'une quelconque des revendications 7 à 9, dans lequel les substituants optionnels sont sélectionnés parmi chloro, fluoro, méthylsulfonyle, éthylsulfonyle, carbamate, méthylcarbamate, méthylaminosulfonyle, éthylaminosulfonyle, méthylsulfonylamino, éthylsulfonylamino, morpholin-1-ylsulfonyle, pipéridin-1-ylsulfonyle, pipérazin-1-ylsulfonyle, 4-méthylpipérazin-1-ylsulfonyle et tétrahydropyrrol-1-ylsulfonyle.

11. Composé selon l'une quelconque des revendications précédentes, dans lequel X est -CH₂-, -S(O)-, -NHSO₂- ou -SO₂NH-.

12. Composé selon l'une quelconque des revendications 1 à 10, dans lequel X est -S- ou -SO₂-.

13. Composé selon l'une quelconque des revendications précédentes, dans lequel R₆ est hydrogène, éthyle ou trifluorométhyle.

14. Composé selon l'une quelconque des revendications 1 à 12, dans lequel R₆ est méthyle.

15. Composé selon l'une quelconque des revendications précédentes, dans lequel R₇ et R₈ sont chacun hydrogène.

16. Composé selon l'une quelconque des revendications 1 à 14, dans lequel l'un de R₇ et R₈ est méthyle et l'autre hydrogène.

17. Composé selon l'une quelconque des revendications 1 à 14, dans lequel R₇ et R₈ pris ensemble avec l'atome de carbone auquel ils sont attachés forment un noyau cyclopropyle, cyclopentyle ou cyclohexyle.

18. Composé selon la revendication 1 ou 2, dans lequel R₁, R₂, R₃ et R₄ sont indépendamment sélectionnés parmi hydrogène, chloro, fluoro, cyano, méthyle trifluorométhyle, méthoxy, et trifluorométhoxy ; X est -S- ou -SO₂-; R₅ est phényle optionnellement substitué, R₆ est méthyle, et R₇ et R₈ sont hydrogène.

19. Composé selon la revendication 18, dans lequel R₅ est phényle ou phényle substitué par un ou deux substituants sélectionné(s) parmi chloro, fluoro, trifluorométhyle, méthylsulfonyle, éthylsulfonyle, carbamate, méthylcarbamate, méthylaminosulfonyle, éthylaminosulfonyle, méthylsulfonylamino, éthylsulfonylamino, morpholin-1-ylsulfonyle, pipéridin-1-ylsulfonyle, pipérizin-1-ylsulfonyle, 4-méthylpipérazin-1-ylsulfonyle et tétrahydropyrrol-1-ylsulfonyle.

20. Composé pharmaceutique comprenant un composé selon l'une quelconque des revendications 1 à 19 ainsi qu'un vecteur de qualité pharmaceutique.

21. Composé selon l'une quelconque des revendications 1 à 19, pour la fabrication d'une composition servant au traitement de : asthme, maladie pulmonaire obstructive chronique, rhinite, syndrome allergique des voies respiratoires ou rhino-bronchite allergique.
